# EUROPEAN PATENT APPLICATION

(11) **EP 2 471 910 A2**
(43) Date of publication of application: **04.07.2012**
(21) Application number: 10794355.7
(22) Date of filing: 30.06.2010
(51) Int. Cl.: C12N 9/00, C12N 15/52, C12P 7/56

(54) **PREPARATION METHOD OF LACTATE POLYMERS AND LACTATE COPOLYMERS USING POLYHYDROXYALKANOATE SYNTHASE MUTANTS**

(30) Priority: 30.06.2009 KR 20090059488
(71) Applicant: LG Chem, Ltd., Youngdungpo-gu Seoul 150-721 (KR); Korea Advanced Institute of Science and Technology, Daejeon-si 305-701 (KR)
(72) Inventor: YANG, Taek Ho, Daejeon 305-707 (KR); KANG, Hye Ok, Daejeon 302-763 (KR); LEE, Sang Yup, Daejeon 305-761 (KR); JUNG, Yu Kyung, Daejeon 305-701 (KR)
(74) Representative: Goddar, Heinz J.
(86) International application number: PCT/KR2010/004240
(87) International publication number: WO 2011/002220

(57) **Abstract**

Mutants of various polyhydroxyalkanoate (PHA) synthases capable of synthesizing a lactate polymer (PLA) and a lactate copolymer (PLA copolymer), and a method of preparing a lactate polymer and a lactate copolymer using the same are provided. More specifically, a mutant of polyhydroxyalkanoate synthase set forth in SEQ ID NO: 2, 4, 6, or 8, and a method of preparing lactate polymer and lactate copolymer using the mutant of synthase are provided. The polyhydroxyalkanoate synthase set forth in SEQ ID NO: 2, 4, 6, or 8 can have an activity of synthesizing a lactate polymer and a lactate copolymer by an amino acid sequence mutation affecting an activity of synthesizing a lactate polymer, and can produce a lactate polymer and a copolymer that have different features, respectively, by using the mutants of the synthase.

## Description

### Field of the Invention

The present invention relates to mutants of various polyhydroxyalkanoate synthases capable of synthesizing a lactate polymer and a lactate copolymer using lactyl-CoA as a substrate. Also, the present invention relates to a method of preparing a lactate polymer and a lactate copolymer using mutants of polyhydroxyalkanoate synthases having an increased activity of synthesizing a lactate polymer and a lactate copolymer.

### Discussion of Related Art

Polylactate (PLA) is a typical biodegradable polymer derived from lactate and having high applicability as a polymer for medical use or a general-purpose polymer. PLA is now being prepared by polymerization of lactate produced from microorganism fermentation, but only PLA with low molecular weight (1000-5000 Dalton) is produced by direct polymerization of lactate. There is a method of polymerizing PLA with high molecular weight using a chain coupling agent from PLA with low molecular weight obtained from direct polymerization of lactate in order to synthesize PLA with at least 100,000 daltons. However, the above-mentioned method has disadvantages in that adding an organic solvent and the chain coupling agent makes the process more complex and they are also difficult to remove. As a process of producing PLA with high molecular weight that is now commercialized, there is a method of synthesizing PLA through a ring-opening condensation reaction of a lactide ring after converting lactate into lactide.

When PLA is synthesized from lactate through a chemical synthesis, PLA homopolymer can be easily obtained, but synthesis of PLA copolymer having various monomer compositions is difficult and has very low industrial efficiency.

Meanwhile, polyhydroxyalkanoate (PHA) is a polyester, which is accumulated inside a microorganism as energy or a reserve material of a carbon source when there is an excessive carbon source, but there is a lack of other nutrients, such as phosphorus, nitrogen, magnesium, oxygen, and the like. Since PHA has the similar physical properties as a synthetic polymer derived from conventional petroleum, and also has complete biodegradability, it is recognized as an alternative material of the conventional synthetic plastic.

An enzyme that converts metabolites of a microorganism into PHA monomer and PHA synthase, which synthesizes PHA polymer using PHA monomer, is required to produce PHA in a microorganism. Also, the same system is required to synthesize PLA and PLA copolymers using a microorganism, and an enzyme capable of further providing lactyl-CoA is also required in addition to the enzyme capable of providing hydroxyacyl-CoA, which is a substrate of original PHA synthase.

That is, this shows that the introduction of a monomer-supplying enzyme capable of smoothly supplying lactyl-CoA by expressing an activated type without inhibiting cell growth and PHA synthase capable of effectively recognizing lactyl-CoA as a substrate is very important in order to effectively produce PLA and PLA copolymer using a microorganism.

### SUMMARY OF THE INVENTION

Accordingly, the present inventors investigated polyhydroxyalkanoate synthases with high homology of an amino acid sequence with polyhydroxyalkanoate synthase derived from *Pseudomonas* sp. 6-19 that has been used for the conventional system. Then, the present inventors confirmed that a lactate polymer and a copolymer thereof can be produced with high efficiency by cloning polyhydroxyalkanoate synthase from four typical *Pseudomonas* strains among the synthases with high homology and then preparing mutants that change amino acid sequences affecting a synthesis activity of the lactate polymer and lactate copolymer. Therefore, the present invention was completed based on these facts.

An object of the present invention is to provide a recombinant microorganism capable of producing a lactate polymer or copolymer thereof using the mutants of various polyhydroxyalkanoate synthases and a method of preparing a lactate polymer or a copolymer thereof using the recombinant microorganism.

In order to achieve the object, the present invention provides a mutant of polyhydroxyalkanoate synthase that synthesizes a lactate polymer or a lactate copolymer using lactyl-CoA as a substrate, in which the mutant has an amino acid sequence comprising at least one mutation selected from the group consisting of

a) E130D and Q481K;

b) E130D, S325T, and Q481K;

c) E130D, S477F, and Q481K;

d) E130D, S325T, S477F, and Q481K; and

e) E130D, S325T, S477G, and Q481K;

in an amino acid sequence of polyhydroxyalkanoate synthase that is set forth in SEQ ID NO: 2, 4, 6, or 8.

In addition, the present invention provides a gene encoding the mutant of polyhydroxyalkanoate synthase, a recombinant vector for a synthesis of a lactate polymer or a lactate copolymer comprising the gene, a transformant transformed with the recombinant vector, and a method of preparing a lactate polymer or a lactate copolymer comprising culturing the transformant.

### BRIEF DESCRIPTION OF THE DRAWINGS

The above and other objects, features and advantages of the present invention will become more apparent to those of ordinary skill in the art by describing in detail exemplary embodiments thereof with reference to the attached drawings, in which:

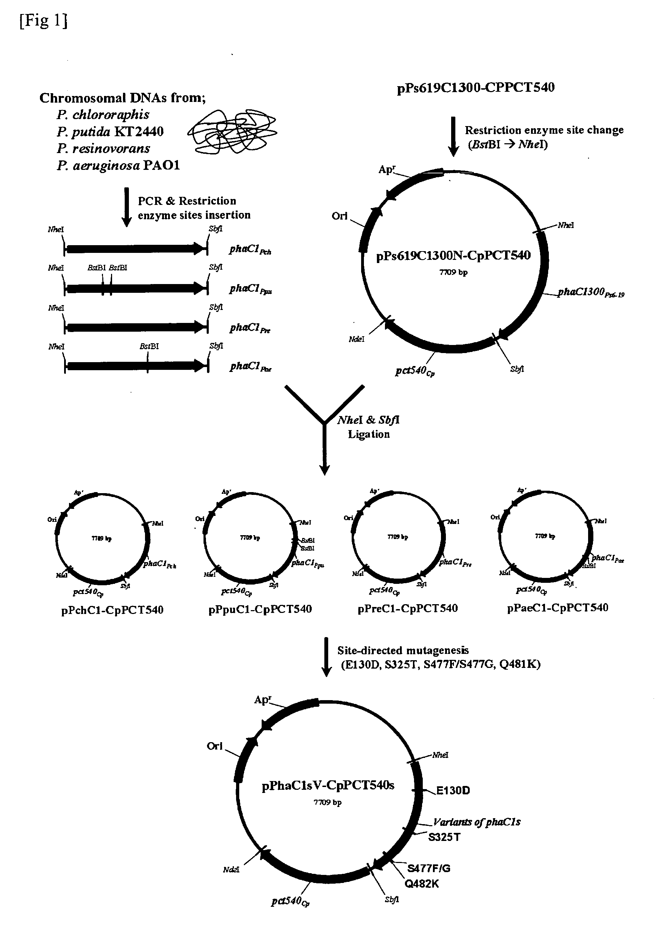

Fig. 1 shows a diagram of a process of constructing a recombinant expression vector comprising Type II polyhydroxyalkanoate synthases having high amino acid sequence homology with polyhydroxyalkanoate synthase derived from *Pseudomonas* sp. MBEL 6-19 of the present invention and propionyl-CoA transferase mutant (Pct540Cp) derived from *Clostridium propionicum,* and mutants of polyhydroxyalkanoate synthases with increased activity of synthesizing a lactate polymer and a lactate copolymer from the recombinant expression vector; and

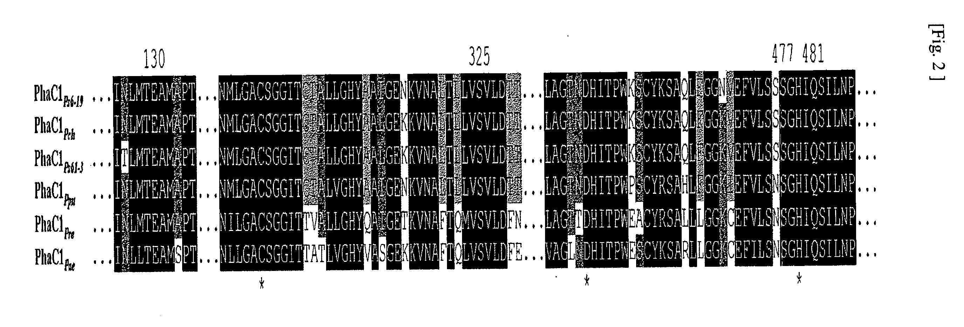

Fig. 2 shows multiple alignments of amino acid sequences of the polyhydroxyalkanoate synthases derived from *Pseudomonas* sp. MBEL 6-19 used for the present invention, a polyhydroxyalkanoate synthase derived from *Pseudomonas chlororaphis,* a polyhydroxyalkanoate synthase derived from *Pseudomonas putida* KT2440, a polyhydroxyalkanoate synthase derived from *Pseudomonas resinovorans,* and a polyhydroxyalkanoate synthase derived from *Pseudomonas aeruginosa* PAO1, in which amino acid residues (Cys296, Asp451, His479) that are presumed to be catalytic residues are marked with "*" and amino acid residues (Glu130, Ser325, Ser477, Glen481) affecting a change of substrate specificity to lactyl-CoA are marked respectively with the numbers in the amino acid sequence.

### DETAILED DESCRIPTION OF EXEMPLARY EMBODIMENTS

The present invention provides a recombinant microorganism having an ability to produce a lactate polymer and a copolymer thereof, by comprising a lactyl-CoA supply enzyme gene, and a polyhydroxyalkanoate synthase gene derived from *Pseudononas chlororaphis* (SEQ ID NO: 1), a polyhydroxyalkanoate synthase gene derived from *Pseudomonas putida* KT2440 (SEQ ID NO: 3), a polyhydroxyalkanoate synthase gene derived from *Pseudomonas resinovorans* (SEQ ID NO: 5), or a polyhydroxyalkanote synthase gene derived from *Pseudomonas aeruginosa* PA01 (SEQ ID NO: 7) at the same time.

The present invention provides a mutant of a polyhydroxyalkanoate synthase that synthesizes a lactate polymer or a lactate copolymer using lactyl-CoA as a substrate, in which the mutant has an amino acid sequence comprising at least one selected from the group consisting of

a) E130D and Q481K;

b) E130D, S325T, and Q481K;

c) E130D, S477F, and Q481K;

d) E130D, S325T, S477F, and Q481K; or

e) E130D, S325T, S477G, and Q481K;

in amino acid sequences of polyhydroxyalkanoate synthase that are set forth in SEQ ID NO: 2, 4, 6, or 8.

The E130D mutation refers to a mutation in which the 130^{th} amino acid glutamate is substituted with aspartic acid; the S325T mutation refers to a mutation in which the 325^{th} amino acid serine is substituted with threonine; the S477F mutation refers to a mutation in which the 477^{th} amino acid serine is substituted with phenylalanine; The Q481K mutation refers to a mutation in which the 481^{st} amino acid glutamine is substituted with lysine; and the S477G mutation refers to a mutation in which the 477^{th} amino acid serine is substituted with glycine. An activity of producing a lactate polymer or a lactate copolymer by the mutant of polyhydroxyalkanoate synthase may be newly formed or increased due to the mutations in the amino acid sequence as mentioned above.

The amino acid sequences of SEQ ID NOS: 2, 4, 6, and 8 refer to an amino acid sequence of polyhydroxyalkanoate synthase derived from *P. chlororaphis,* an amino acid sequence of polyhydroxyalkanoate synthase derived from *P. putida* KT2440, an amino acid sequence of polyhydroxyalkanoate synthase derived from *P*. *resinovorans,* and an amino acid sequence of polyhydroxyalkanoate synthase derived from *P. aeruginosa* PA01, respectively. The present inventors confirmed that the lactate polymer and the lactate copolymer can be produced with high efficiency using lactyl-CoA as a substrate when using the mutants of PHA synthases derived from the above-described four *Pseudomonas* strains. Therefore, the present invention was completed based on these facts.

Also, the present invention provides genes that encode the mutants of the polyhydroxyalkanoate synthases.

Also, the present invention provides a recombinant vector for synthesis of a lactate polymer or a copolymer, comprising the genes.

Also, for the present invention, the recombinant vector further includes a propionyl-CoA transferase gene derived from *Clostridium propionicum*(pct_{Cp}) of SEQ ID NO: 77. The propionyl-CoA transferase is an enzyme that converts lactate and 3-hydroxybutyrate into lactyl-CoA and 3-hydroxybutyrate-CoA, respectively.

Preferably, the propionyl-CoA transferase gene may include a mutant gene of propionyl CoA-transferase comprising at least one of mutation selected from the group consisting of

a) A1200G mutation in a base sequence of SEQ ID NO: 77;

b) T78C, T669C, A1125G, and T1158C mutations in a base sequence of SEQ ID NO: 77;

c) Gly335Asp mutation in an amino acid sequence of SEQ ID NO: 78 andA1200G mutation in a base sequence of SEQ ID NO: 77;

d) Ala243Thr mutation in an amino acid sequence of SEQ ID NO: 78 and A1200G mutation in a base sequence of SEQ ID NO: 77;

e) Asp65Gly mutation in an amino acid sequence of SEQ ID NO: 78 and T669C, A1125G, and T1158C mutations in a base sequence of SEQ ID NO: 77;

f) Asp257Asn mutation in an amino acid sequence of SEQ ID NO: 78 and A1200G mutation in a base sequence of SEQ ID NO: 77;

g) Asp65Asn mutation in an amino acid sequence of SEQ ID NO: 78 and T699C, A1125G, and T1158C mutations in a base sequence of SEQ ID NO: 77;

h) Thr199Ile mutation in an amino acid sequence of SEQ ID NO: 78 and T699C, A1125G, and T1159C mutations in a base sequence of SEQ ID NO: 77; and

i) Va1193A1a mutation in an amino acid sequence of SEQ ID NO: 78 and T78C, T699C, and T1158C mutations in a base sequence of SEQ ID NO: 77:

In the base sequence of SEQ ID NO: 77, the A1200G mutation refers to a mutation in which the 1200^{th} base adenine is substituted with guanine; the T78C mutation refers to a mutation in which the 78^{th} base thymine is substituted with cytosine; the T669C mutation refers to a mutation in which the 669th base thymine is substituted with cytosine; the A1125G mutation refers to a mutation in which the 1125^{th} base adenine is substituted with guanine; and the T1158C mutation refers to a mutation in which the 1158^{th} base thymine is substituted with cytosine. In the amino acid sequence of SEQ ID NO: 78, the Gly335Asp mutation refers to a mutation in which the 335^{th} amino acid glycine is substituted with aspartic acid; the Ala243Thr mutation refers to a mutation in which the 257^{th} amino acid alanine is substituted with threonine; the Asp65Asn mutation refers to a mutation in which the 65^{th} amino acid aspartic acid is substituted with asparagine; the Thr199Ile mutation refers to a mutation in which the 199^{th} amino acid threonine is substituted with isoleucine; and the Va1193Ala mutation refers to a mutation in which the 193^{th} amino acid valine is substituted with alanine.

Preferably, the mutant gene of the propionyl CoA-transferase may be the mutant gene of the propionyl CoA-transferase (pct 540Cp) including the Va1193Ala mutation in an amino acid sequence of SEQ ID NO: 78 and the T78C, T699C, and T1158C mutation in a base sequence of SEQ ID NO: 77.

In addition, the present invention provides a transformant transformed with any one among the recombinant vectors, and a transformant obtained by a transformation of the transformed strains without the propionyl-CoA transferase using any one of the above-mentioned recombinant vectors is included in the scope of the present invention.

The recombinant vector according to the present invention may be transformed into a proper host cell using a general method. Bacteria, yeast, fungi, and the like may be used as a host cell, but the present invention is not limited thereto. The preferred host cell according to the present invention is a prokaryotic cell, and preferably, *E. coli.* Examples of a suitable prokaryotic cells include *E. coli* DH5a, *E. coli* JM101, *E. coli* K12 294, *E. coli* W3110, *E. coli* X1776, *E. coli* XL-1Blue (Stratagene), *E. coli* B, and the like. However, an *E. coli* strain such as FMB101, NM522, NM538, and NM539, and prokaryotic cell of other species and genera may be also used. In addition to the above-described *E. coli, Agrobacterium* genus strains, such as *Agrobacterium* A4, *Bacilli,* such as *Bacillus subtilis,* another enterbacter, such as *Salmonella typhimurium* or *Serratia marcescens,* and various *Pseudomonas* genus strains may be used as a host cell, but the present invention is not limited thereto.

A target plant for a transformation that can be used for the present invention may be tobacco, a tomato, a chili, a bean, rice, corn, and the like, but the present invention is not limited thereto. In addition, even though the plant used for a transformation is a plant of sexual propagation, it can be understood by a person of ordinary skill in the art that the plant can be repeatedly reproduced nonsexually by a tissue culture, and the like.

Also, the present invention provides a method of preparing a lactate polymer or a copolymer thereof, comprising culturing the transformant.

More preferably, the present invention provides a method of preparing a lactate polymer or a lactate copolymer, in which the culturing is performed under an environment of containing hydroxyalkanoate, and the prepared copolymer is hydroxyalkanoate-co-3-lactate that is the copolymer comprising a hydroxyalkanoate monomer unit and a lactate monomer unit.

In the present invention, the copolymer refers to a dipolymer having two types of monomers, a terpolymer having three types of monomers, a tetrapolymer having four types of monomers, and the like.

In the present invention, the hydroxyalkanoate may be at least one selected from the group consisting of 3-hydroxybutyrate, 3-hydroxyvalerate, 4-hydroxybutyrate, (D)-3-hydroxycarboxylic acids with a medium chain length of 6 to 14 carbon numbers, 3-hydroxypropionic acid, 3-hydroxyhexanic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytetradecanoic acid, 3-hydroxyhexadecanoic acid, 4-hydroxyvaleric acid, 4-hydroxyhexanoic acid, 4-hydroxyheptanoic acid, 4-hydroxyoctanoic acid, 4-hydroxydecanoic acid, 5-hydroxyvaleric acid, 5-hydroxyhexanoic acid, 6-hydroxydodecanoic acid, 3-hydroxy-4-pentenoic acid, 3-hydroxy-4-trans-hexenoic acid, 3-hydroxy-4-cis-hexenoic acid, 3-hydroxy-5-hyxenoic acid, 3-hydroxy-6-trans-octenoic acid, 3-hydroxy-6-cis octenoic acid, 3-hydroxy-7-octenoic acid, 3-hydroxy-8-nonenoic acid, 3-hydroxy-9-decenoic acid, 3-hydroxy-5-cis-dodecenoic acid, 3-hydroxy-5-cis-dodecenoic acid, 3-hydroxy-5-cis-tetradecenoic acid, 3-hydroxy-7-cis-tetradecenoic acid, 3-hydroxy-5,8-cis-cis-tetradecenoic acid, 3-hydroxy-4-methylvaleric acid, 3-hydroxy-4-methylhexanoic acid, 3-hydroxy-5-methylhexanoic acid, 3-hydroxy-6-methylhexanoic acid, 3-hydroxy-4-methyloctanoic acid, 3-hydroxy-5-methyloctanoic acid, 3-hydroxy-6-methyloctanoic acid, 3-hydroxy-7-methyloctanoic acid, 3-hydroxy-6-methylnonanoic acid, 3-hydroxy-7-methylnonanoic acid, 3-hydroxy-8-methylnonanoic acid, 3-hydroxy-7-methyldecanoic acid, 3-hydroxy-9-methyldecanoic acid, 3-hydroxy-7-methyl-6-octenoic acid, malic acid, 3-hydroxysuccinic acid-methylester, 3-hydroxyadipinic acid-methylester, 3-hydroxysuberic acid-methylester, 3-hydroxyazelaic acid-methylester, 3-hydroxysebacic acid-methylester, 3-hydroxysuberic acid-ethylester, 3-hydroxysebacic acid-ethylester, 3-hydroxypimelic acid-propylester, 3-hydroxysebacic acid-benzylester, 3-hydroxy-8-acetoxyoctanoic acid, 3-hydroxy-9-acetoxynonanoic acid, phenoxy-3-hydroxybutyric acid, phenoxy-3-hydroxyvaleric acid, phenoxy-3-hydroxyheptanoic acid, phenoxy-3-hydroxyoctanoic acid, para-cyanophenoxy-3-hydroxybutyric acid, para-cyanophenoxy-3-hydroxyvaleric acid, para-cyanophenoxy-3-hydroxyhexanoic acid, para-nitrophenoxy-3-hydroxyhexanoic acid, 3-hydroxy-5-phenylvaleric acid, 3-hydroxy-5-cyclohexylbutyric acid, 3,12-dihydroxydodecanoic acid, 3,8-dihydroxy-5-cis-tetradecenoic acid, 3-hydroxy-4,5-epoxydecanoic acid, 3-hydroxy-6,7-epoxydodecanoic acid, 3-hydroxy-8,9-epoxy-5,6-cis-tetradecanoic acid, 7-cyano-3-hydroxyheptanoic acid, 7-cyano-3-hydroxyheptanoic acid, 3-hydroxy-7-fluoroheptanoic acid, 3-hydroxy-9-fluorononanoic acid, 3-hydroxy-6-chlorohexanoic acid, 3-hydroxy-8-chlorooctanoic acid, 3-hydroxy-6-bromohexanoic acid, 3-hydroxy-8-bromooctanoic acid, 3-hydroxy-11-bromoundecanoic acid, 3-hydroxy-2-butenoic acid, 6-hydroxy-3-dodecenoic acid, 3-hydroxy-2-methylbutyric acid, 3-hydroxy-2-methylvaleric acid and 3-hydroxy-2,6-dimethyl-5-heptenoic acid.

For the present invention, the term "vector" refers to a DNA construct comprising a DNA sequence to be operably linked to a suitable control sequence that can express DNA inside a host. A vector may be a plasmid, a phage particle, or simply a latent genomic insert. When a vector is transformed into a suitable host, it may be replicated or functioned regardless of a host genome, or in some cases, it may be integrated into a genome itself. A plasmid is the type that is most generally used as a vector, and thus plasmid and vector are sometimes used interchangeably in the present invention. However, the present invention also includes other types of a vector having the same function as the function that is known or is to be known in the art.

The expression "expression control sequence" refers to a DNA sequence that is essential for expression of a coding sequence that is operably linked in a specific host organism. The control sequence includes a promoter for performing a transcription, any operator sequence for controlling the transcription, a sequence for encoding a suitable mRNA ribosome binding domain, and a sequence for controlling terminations of the transcription and translation. For example, the control sequence suitable for a prokaryotic cell includes a promoter, any operator sequence, and a ribosome binding domain. In the eukaryotic cell, the control sequence includes a promoter, a polyadenylated signal, and an enhancer. A factor that has the biggest impact on the expression level of gene in a plasmid is a promoter. An SRa promoter, a promoter derived from cytomegalovirus, and the like are preferably used as a promoter for high expression.

Any one of very various expression control sequences may be used as a vector in order to express a DNA sequence of the present invention. Examples of the useful expression control sequence include, for example, an initial promoter and a late promoter of SV40 or adenovirus, a lac system, a trp system, a TAC or TRC system, T3 and T7 promoters, a major operator of phage lambda and a promoter region, a control region of fd coding protein, promoters to 3-phosphoglycerate kinase or other glycol degradation enzymes, the promoters of the phosphase, for example, Pho5, a promoter of a yeast alpha-hybrid, and other sequences for an inducement and a constitution that are known for controlling gene expression of a prokaryotic cell or a eukaryotic cell, or viruses thereof, and combinations thereof.

Nucleic acid is operably linked when it is arranged with a functional relationship with other nucleic acid sequences. It may be a gene and control sequence(s) that is linked in a process that enables the gene expression when a proper molecule (for example, transcriptional activation protein) is linked to the control sequence(s). For example, DNA for a pre-sequence or a secretion leader is operably linked to DNA for a polypeptide when expressing a pre-protein participating in secretion of a polypeptide; a promoter or an enhancer is operably linked to a coding sequence when affecting transcription of a sequence; a ribosome binding domain is operably linked to a coding sequence when affecting transcription of a sequence; or a ribosome binding domain is operably linked to a coding sequence when it is arranged to be easily translated. Generally, "operably linked" refers to a contact of a linked DNA sequence, or that the secretion leader is contacted and presented in the leading frame. However, the enhancer is not required to contact. When the domain is not presented, a synthetic oligonucleotide adaptor or linker according to a general method is used as mentioned above.

The term "expression vector" used for the present invention generally refers to a double-strained DNA fragment as a general recombinant carrier inserted with a xenogeneic DNA fragment. Here, xenogeneic DNA refers to heterogeneous DNA that is natively undiscovered DNA in a host cell. The expression vector is inside the host cell, can be replicated regardless of host chromosome DNA, and may produce several copies of a vector and (xenogeneic) DNA inserted in the same.

As is known in the art, the relevant gene is operably linked to the transcription and translation expression control sequences that function inside a selected expression host in order to increase the expression level of a transformed gene in the host cell. Preferably, the expression control sequence and relevant gene are included in one expression vector comprising the bacteria selection marker and replication origin together. When the expression host is a eukaryotic cell, the expression vector should further include a useful expression marker in a eukaryotic expression host.

In the present invention, the recombinant vector may be various vectors comprising a plasmid vector, a bacteriophage vector, a cosmid vector, and a yeast artificial chromosome (YAC) vector. The plasmid vector is preferably used for the object of the present invention. A typical plasmid vector that can be used for the object has a structure comprising (a) a replication origin that allows a replication to be effectively performed to include hundreds of plasmid vectors per host cell, (b) an antibiotic-resistance gene that allows a host cell transformed with a plasmid vector to be selected, and (c) a restriction site of restriction enzyme that can be inserted with a foreign DNA fragment. Even if there is no suitable restriction site of a restriction enzyme, a vector and foreign DNA may be easily ligated when using the linker and the synthetic oligonuclerotide adaptor according to a general method.

In addition, the transformation of a prokaryotic cell may be easily achieved using a calcium chloride method as described in section 1.82 of Sambrook *et al.,* supra. Optionally, electroporation (Neumann et al., EMBO J., 1: 841(1982)) may also be used for the transformation of the cells.

A transfection of a plant for preparing the plant comprising the gene of the converting enzyme and the gene of the synthase of the present invention may be achieved by a general method using *Agrobacterium,* a virus vector, and the like. For example, the *Agrobacterium* genus microorganism is transformed with the recombinant vector comprising the gene according to the present invention, and then the transformed *Agrobacterium* genus microorganism is transfected into a tissue, and the like of the target plant to obtain a transfected plant. More specifically, the transfected plant may be prepared by the steps of (a) transfecting a target plant by preculturing an explant of the target plant, and then co-culturing it with the transformed *Agrobacterium;* (b) obtaining callus by culturing the transfected explant in a callus induction medium; and (c) developing a shoot by cutting the obtained callus and then culturing it in a shoot induction medium.

The term "explant" in the present invention refers to a fragment of tissue cut from a plant, and includes cotyledon or hypocotyl. The explant used for the method of the present invention may be cotyledon or hypocotyl, and the cotyledon obtained through germinating in an MS medium after disinfecting and washing a plant seed is more preferably used.

Hereinafter, the present invention will be described in further detail with reference to Examples. Examples are only for illustrating more specifically, and the range of the present invention is not limited to Examples.

Especially, the following Examples disclose only synthesis of poly(3-hydroxybutyrate-co-lactate) (P(3HB-*co*-LA)) by adding 3-hydroxybutyrate (3-HB) to a synthesis of lactate copolymer using a mutant of Type II PHA synthase, but it is obvious to a person of ordinary skill in the art that the copolymer of the polyhydroxyalkanoate and lactate can be produced by adding polyhydroxyalkanoate in addition to 3-HB.

**<Example 1> Gene Cloning and Investigation of PHA Synthase with High Homology with Amino Acid Sequence of PHA Synthase Derived from *Pseudomonas* sp. MBEL 6-19**

In order to investigate polyhydroxyalkanoate synthase with high homology of an amino acid sequence with polyhydroxyalkanoate synthase derived from *Pseudomonas* sp. MBEL 6-19 (KCTC 11027BP) (PhaC1_{*Ps6*-*19*}) used for the present invention, Basic Local Alignment Search Tool (BLAST) analysis provided by the National Center for Biotechnology Information (NCBI) was used, and the synthases showing a relatively high amino acid sequence homology among the results are shown in Table 1. All of the enzymes were included in the group of Type II polyhydroxyalkanoate synthase that is medium-chain-length (MCL)-PHA synthase for polymerization of the substrate with a relatively long carbon chain.

**[Table 1]**

| Polyhydroxyalkanoate synthase showing high homology of amino acid sequence with PhaC1*_{Ps6-19}* | | | |
|---|---|---|---|
| Organism | Nucleotide identity | Amino acid identity | Genbank accession no. |
| *Pseudomonas* sp. MBEL 6-19 | 100 | 100 | FJ626663 |
| *Pseudomonas* sp. 3Y2 | 95 | 97 | AY754343 |
| *Pseudomonas fluorescens* PfO-1 | 88 | 93 | CP000094 |
| *Pseudomonas* sp. KBOS 03 | 87 | 91 | AY790327 |
| *Pseudomonas chlororaphis* | *85* | *90* | *AB049413* |
| *Pseudomonas corrugata* CFBP5454 | 85 | 89 | AY910767 |
| *Pseudomonas* sp. 61-3 | 84 | 89 | AB014758 |
| *Pseudomonas fluorescens* Pf-5 | 85 | 89 | CP000076 |
| *Comamonas testosteroni* | *83* | *87* | *AY790326* |
| *Burkholderia caryophylli* | 82 | 86 | *AF394660* |
| *Aeromonas hydrophila* | *80* | *82* | *AY786298* |
| *Pseudomonas putida* KT2440 | 80 | 81 | AE015451 |
| *Pseudomonas pseudoalcaligenes* HBQ06 | 80 | 79 | AF336848 |
| *Pseudomonas resinovorans* | *80* | *81* | *AF129396* |
| *Pseudomonas* sp. HJ-2 | 80 | 78 | AY370934 |
| *Pseudomonas stutzeri* 1317 | 79 | 79 | AY278219 |
| *Pseudomonas aeruginosa* PAO1 | 78 | 77 | AE004091 |

The following four typical polyhydroxyalkanoate synthases were selected for an experiment in order to implement the present invention among the polyhydroxyalkanoate synthases [*P. chlororaphis* (KCTC 12349), *P. putida* KT2440 (ATCC 47054), *P. resinovorans* (KCTC 12498), and *P. aeruginosa* PAO1 (KCTC 1637)]. The whole DNA was extracted from each *Pseudomonas* strain in order to isolate the gene of polyhydroxyalkanoate synthase used for the present invention, and cloned with PCR by designing a primer as shown in Table 2 based on the synthase gene sequences deposited in NCBI Genbank.

**[Table 2]**

| Primers for Cloning Polyhydroxyalkanoate Synthases | | | |
|---|---|---|---|
| Synthase | Microorganism | Synthase Cloning Primer | Recognition Site Insert Primer |
| PhaC1*_{Pch}* | *P. chlororaphis* | SEQ ID NOS: 11 & 12 | SEQ ID NOS: 9&10, 13&26 |
| PhaC1*_{Ppu}* | *P. putida* KT2440 | SEQ ID NOS: 15 & 16 | SEQ ID NOS: 9&14, 17&26 |
| PhaC1*_{Pre}* | *P. resinovorans* | Sequence Nos.*19* & *20* | SEQ ID NOS: *9&18,21&26* |
| *PhaC1_{Pae},* | *P. aeruginosa* PAO1 | SEQ ID NOS: 23 & 24 | SEQ ID NOS: 9&22, 25&26 |

SEQ ID NO: 9: 5' - gca atg ccc gga gcc ggg cta gct ag - 3'

SEQ ID NO: 10: 5' - gtc atc gtt att ctt gtt act cat gat ttg att gtc tct ctg - 3'

SEQ ID NO: 11: 5' - Cag aga gac aat caa atc atg agt aac aag aat aac gat
gac - 3'

SEQ ID NO: 12: 5' - gca ctc atg caa gcg tta acg ttc atg gac ata agt acc -
3'

SEQ ID NO: 13: 5' - ggt act tat gtc cat gaa cgt taa cgc ttg cat gag tgc - 3'

SEQ ID NO: 14: 5' - ctc atc gtt gtt ctt gtt act cat gat ttg att gtc tct ctg - 3'

SEQ ID NO: 15: 5’ - cag aga gac aat caa atc atg agt aac aag aac aac gat
gag - 3’

SEQ ID NO: 16: 5' - gca ctc atg caa gcg tca acg ctc gtg aac gta ggt g - 3'

SEQ ID NO: 17: 5' - cac cta cgt tca cga gcg ttg acg ctt gca tga gtg c - 3'

SEQ ID NO: 18: 5' - gtc ttc att gtt ctt gtt get cat gat ttg att gtc tct ctg - 3'

SEQ ID NO: 19: 5' - cag aga gac aat caa atc atg agc aac aag aac aat gaa
gac - 3'

SEQ ID NO: 20: 5' - gca ctc atg caa gcg tca tcg ctc gtg cac ata ggt g - 3'

SEQ ID NO: 21: 5' - cac cta tgt gca cga gcg atg acg ctt gca tga gtg c - 3'

SEQ ID NO: 22: 5' - ctc gtt att gtt ctt ctg act cat gat ttg att gtc tct ctg - 3'

SEQ ID NO: 23: 5' - cag aga gac aat caa atc atg agt cag aag aac aat aac
gag - 3'

SEQ ID NO: 24: 5' - gca ctc atg caa gcg tca tcg ttc atg cac gta ggt tc- 3'

SEQ ID NO: 25: 5' - gaa cct acg tgc atg aac gat gac gct tgc atg agt gc - 3'

SEQ ID NO: 26: 5' - gaa att gtt atc cgc ctg cag g - 3'

As a result of agarose gel electrophorosis of a PCR reactant, it was found that a gene fragment of 1.7 kbp size corresponding to the polyhydroxyalkanoate synthase gene was confirmed. For the expression of each synthase, a constitutive expression system was introduced, in which the system was in an operon type to express along with a mutant gene (*pct540_{Cp}*) of propionyl-CoA transferase derived from C. *propionicum* that is a monomer supply enzyme. Prior to this, it was found from the gene sequence of polyhydroxyalkanoate synthase deposited in NCBI Genbank that many *BstB*I sites used for a cloning site in the constitutive expression system in the conventional operon type were included in some polyhydroxyalkanoate synthase genes that were newly cloned. In order to solve the above problem, a pPs619C1300N-CpPCT540 vector in which a *BstB*I site was converted into an *Nhe*I site that was a unique cloning site was prepared based on pPs619C1300-CpPCT540 (WO09/022797) using a site-directed mutagenesis (SDM) method *(see* Fig. 1.).

The prepared pPs619C1300N-CpPCT540 vector was cleaved with *Nhe*I/*Sbf*I to remove *phaC1300pₛ₆-₁₉,* which is the conventional gene of polyhydroxyalkanoate synthase, and then the four obtained types of synthase genes were inserted to an *Nhe*I*lSbf*I recognition domain using SEQ ID NOS: 11 to 32 to complete each recombinant vector. The four prepared synthase gene PCR reactants were used as a template and an overlapping PCR was performed using "recognition site insert primer" as listed in Table 2 in order to prepare a polyhydroxyalkanoate synthase gene fragment including an RBS region upstream from a initiation codon while one *Nhe*I*lSbf*I recognition site was included one each end.

The base sequence of the synthase gene of the recombinant vector (pPchC1-CpPCT540, pPpuC1-CpPCT540, pPreC1-CpPCT540, pPaeC1-CpPCT540) comprising the four prepared polyhydroxyalkanoate synthase genes was confirmed by DNA sequencing (SEQ ID NOS: 1, 3, 5, and 7), and the amino acid sequences encoded by the same corresponded to SEQ ID NOS: 2, 4, 6, and 8, respectively. In the present invention, the gene sequences of the three synthases other than the polyhydroxyalkanoate synthase (Phac1*_{Pch}*) derived from *P. chlororaphis* among the four newly cloned polyhydroxyalkanoate synthases were equal to the conventional genes deposited in NCBI Genbank *(see* Table 1), and PhaC1*_{Pch}* had several different nucleotide sequences that were thus deposited at Genbank (Accession no. FJ693714).

As a result of performing multiple alignment analysis of an amino acid sequence of polyhydroxyalkanoate synthase PhaC1Ps6-19 derived from *Pseudomonas* sp. 6-19 and amino acid sequences of the four polyhydroxyalkanoate synthases, it was confirmed that all amino acid residues (G1u130, Ser325, Ser477, Glen481) that are reported to affect a change in substrate specificity (WO08/062999) to lactyl-CoA and the amino acid residues (Cys296, Asp451, His479) that are presumed to be catalytic residues were preserved in common *(see* Fig. 2). Also, it was confirmed that the polyhydroxyalkanoate synthase PhaC1Ps61-3 (Matsusaki et al., J. Bacteriol., 1998, 180:6459-6469; Gene sequence homology 84.3%, Amino acid sequence homology 88.7%) derived from *Pseudomonas* sp. strains 61-3 that is not described in Example of the present invention but shows high homology with PhaC1*_{Ps6-19}* also has an increased substrate specificity to lactyl-CoA and the above-mentioned catalytic residues, and high preservation of amino acid residues *(see* Fig. 2).

**<Example 2> Preparation of Mutant with High Activity of Production of Lactate Polymer and Copolymer**

Type II polyhydroxyalkanoate synthase is known as an MCL-PHA synthase that polymerizes the substrate with a relatively long carbon number, and the studies on mutants with an increased activity of synthesis of short-chain-length (SCL)-PHA through researching various mutations were reported (WO08/062999; Takase et al., J. Biochem., 2003, 133:139-145; Takase et al., Biomacromolecules, 2004, 5:480-485; Matsumoto et al., 2005, Biomacromolecules, 6:99-104; Matsumoto et al., 2006, Biomacromolecules, 7:2436-2442).

For the present invention, mutants as shown in the following Table 3 were prepared by introducing mutants of amino acid sequences that affect an activity of synthesis of a lactate polymer and a copolymer as confirmed from the previous invention (WO08/062999) into four newly obtained Type II polyhydroxyalkanoate synthases (PhaC1*_{Pch}*, PhaC1*_{Ppu}*, PhaC1*_{Pre},* PhaC1*_{Pae}*) using an SDM method with the primers of SEQ ID NOS: 27 to 74.

**[Table 3]**

| Mutants of Type II polyhydroxyalkanoate Synthase | | | |
|---|---|---|---|
| PHA Synthase | Recombinant Plasmid | Amino acid Substitution | Primer or Source |
| PhaC1*_{Ps6-19}* | pPs619C1202-CpPCT540 | E130D | WO08/062999 |
| | | Q481K | |
| | pPs619C1310-CpPCT540 | E130D | WO08/062999 |
| | | S477F | |
| | | Q481K | |
| | pPs619C1301-CpPCT540 | E130D | WO08/062999 |
| | | S325T | |
| | | Q481K | |
| | pPs619C1339-CpPCT540 | E130D | WO08/062999 |
| | | S325T | |
| | | S477F | |
| | | Q481K | |
| | pPs619C1337-CpPCT540 | E130D | SEQ ID NOS: 27&28 |
| | | S325T | SEQ ID NOS: 29&30 |
| | | S477G | SEQ ID NOS: 31&32 |
| | | Q481K | SEQ ID NOS: 33&34 |
| PhaC1*_{Pch}* | pPchC1202-CpPCT540 | E130D | SEQ ID NOS: 35&36 |
| | | Q481K | SEQ ID NOS: 37&38 |
| | pPchC1310-CpPCT540 | E130D | SEQ ID NOS: 35&36 |
| | | S477F | SEQ ID NOS: 39&40 |
| | | Q481K | SEQ ID NOS: 37&38 |
| | pPchC1301-CpPCT540 | E130D | SEQ ID NOS: 35&36 |
| | | S325T | SEQ ID NOS: 41&42 |
| | | Q481K | SEQ ID NOS: 37&38 |
| | pPchC1339-CpPCT540 | E130D | SEQ ID NOS: 35&36 |
| | | S325T | SEQ ID NOS: 41&42 |
| | | S477F | SEQ ID NOS: 39&40 |
| | | Q481K | SEQ ID NOS: 37&38 |
| | pPchC1337-CpPCT540 | E130D | SEQ ID NOS: 35&36 |
| | | S325T | SEQ ID NOS: 41&42 |
| | | S477G | SEQ ID NOS: 43&44 |
| | | Q481K | SEQ ID NOS: 37&38 |
| PhaC1 *_{Ppu}* | pPpuC1202-CpPCT540 | E130D | SEQ ID NOS: 45&46 |
| | | Q481K | SEQ ID NOS: 47&48 |
| | pPpuC1310-CpPCT540 | E130D | SEQ ID NOS: 45&46 |
| | | S477F | SEQ ID NOS: 49&50 |
| | | Q481K | SEQ ID NOS: 47&48 |
| | PpuC1301-CpPCT540 | E130D | SEQ ID NOS: 45&46 |
| | | S325T | SEQ ID NOS: 51&52 |
| | | Q481K | SEQ ID NOS: 47&48 |
| | pPpuC1339-CpPCT540 | E130D | SEQ ID NOS: 45&46 |
| | | S325T | SEQ ID NOS: 51&52 |
| | | S477F | SEQ ID NOS: 49&50 |
| | | Q481K | SEQ ID NOS: 47&48 |
| | pPpuC1337-CpPCT540 | E130D | SEQ ID NOS: 45&46 |
| | | S325T | SEQ ID NOS: 51&52 |
| | | S477G | SEQ ID NOS: 53&54 |
| | | Q481K | SEQ ID NOS: 47&48 |
| PhaC1*_{Pre}* | pPreC1202-CpPCT540 | E130D | SEQ ID NOS: 55&56 |
| | | Q481K | SEQ ID NOS: 57&58 |
| | pPreC1310-CpPCT540 | E130D | SEQ ID NOS: 55&56 |
| | | S477F | SEQ ID NOS: 59&60 |
| | | Q481K | SEQ ID NOS: 57&58 |
| | pPreC1301-CpPCT540 | E130D | SEQ ID NOS: 55&56 |
| | | S325T | SEQ ID NOS: 61&62 |
| | | Q481K | SEQ ID NOS: 57&58 |
| | pPreC1339-CpPCT540 | E130D | SEQ ID NOS: 55&56 |
| | | S325T | SEQ ID NOS: 61&62 |
| | | S477F | SEQ ID NOS: 59&60 |
| | | Q481K | SEQ ID NOS: 57&58 |
| | pPreC1337-CpPCT540 | E130D | SEQ ID NOS: 55&56 |
| | | S325T | SEQ ID NOS: 61&62 |
| | | S477G | SEQ ID NOS: 63&64 |
| | | Q481K | SEQ ID NOS: 57&58 |
| PhaC1*_{Pae}* | pPaeC1202-CpPCT540 | E130D | SEQ ID NOS: 65&66 |
| | | Q481K | SEQ ID NOS: 67&68 |
| | pPaeC1310-CpPCT540 | E130D | SEQ ID NOS: 65&66 |
| | | S477F | SEQ ID NOS: 69&70 |
| | | Q481K | SEQ ID NOS: 67&68 |
| | pPaeC1301-CpPCT540 | E130D | SEQ ID NOS: 65&66 |
| | | S325T | SEQ ID NOS: 71&72 |
| | | Q481K | SEQ ID NOS: 67&68 |
| | pPaeC1339-CpPCT540 | E130D | SEQ ID NOS: 65&66 |
| | | S325T | SEQ ID NOS: 71&72 |
| | | S477F. | SEQ ID NOS: 69&70 |
| | | Q481K | SEQ ID NOS: 67&68 |
| | pPaeC1337-CpPCT540 | E130D | SEQ ID NOS: 65&66 |
| | | S325T | SEQ ID NOS: 71&72 |
| | | S477G | SEQ ID NOS: 73&74 |
| | | Q481K | SEQ ID NOS: 67&68 |

SEQ ID NO: 27: 5'- atc aac ctc atg acc gat gcg atg gcg ccg acc - 3'

SEQ ID NO: 28: 5'- ggt cgg cgc cat cgc atc ggt cat gag gtt gat - 3'

SEQ ID NO: 29: 5'- ctg acc ttg ctg gtg acc gtg ctt gat acc acc - 3'

SEQ ID NO: 30: 5'- ggt ggt atc aag cac ggt cac cag caa ggt cag - 3'

SEQ ID NO: 31: 5'- gaa ttc gtg ctg tcg agc ggc ggg cat atc - 3'

SEQ ID NO: 32: 5'- gat atg ccc gcc gct cga cag cac gaa ttc - 3'

SEQ ID NO: 33: 5'- ggg cat atc aaa agc atc ctg aac ccg c - 3'

SEQ ID NO: 34: 5'- gcg ggt tca gga tgc ttt tga tat gcc c - 3'

SEQ ID NO: 35: 5'- atc aac ctg atg acc gat gcc atg gcg ccg acc - 3'

SEQ ID NO: 36: 5'- ggt egg cgc cat ggc atc ggt cat cag gtt gat - 3'

SEQ ID NO: 37: 5'- ggg cat atc aaa age atc ctc aac ccg c - 3'

SEQ ID NO: 38: 5'- gcg ggt tga gga tgc ttt tga tat gcc c - 3'

SEQ ID NO: 39: 5'- gaa ttc gtc ctc tcc agc ttt ggg cat atc - 3'

SEQ ID NO: 40: 5'- gat atg ccc aaa get gga gag gac gaa ttc - 3'

SEQ ID NO: 41: 5'- ctg acc ctg ctg gtc acc gtg ctc gat acc acc - 3'

SEQ ID NO: 42: 5'- ggt ggt atc gag cac ggt gac cag cag ggt cag - 3'

SEQ ID NO: 43: 5'- gaa ttc gtc ctc tcc agc ggc ggg cat atc - 3'

SEQ ID NO: 44: 5'- gat atg ccc gcc get gga gag gac gaa ttc - 3'

SEQ ID NO: 45: 5'- atc aac ctg atg acc gat gcc atg gcg ccg acc - 3'

SEQ ID NO: 46: 5'- ggt egg cgc cat ggc atc ggt cat cag gtt gat - 3'

SEQ ID NO: 47: 5'- ggg cat atc aaa agc atc ctc aac ccg c - 3'

SEQ ID NO: 48: 5'- gcg ggt tga gga tgc ttt tga tat gcc c - 3'

SEQ ID NO: 49: 5'- gaa ttc gta ctg tcc aac ttt ggg cat atc - 3'

SEQ ID NO: 50: 5'- gat atg ccc aaa gtt gga cag tac gaa ttc - 3'

SEQ ID NO: 51: 5'- ctg acc ctg ctg gtc acc gtg ctg gac acc acc - 3'

SEQ ID NO: 52: 5'- ggt ggt gtc cag cac ggt gac cag cag ggt cag - 3'

SEQ ID NO: 53: 5'- gaa ttc gta ctg tcc aac ggc ggg cat ate - 3'

SEQ ID NO: 54: 5'- gat atg ccc gcc gtt gga cag tac gaa ttc - 3'

SEQ ID NO: 55: 5'- atc aac ctg atg acc gat gcg atg gcg ccc acc - 3'

SEQ ID NO: 56: 5'- ggt ggg cgc cat cgc atc ggt cat cag gtt gat - 3'

SEQ ID NO: 57: 5'- ggc cac atc aaa agc att ctc aac cca c - 3'

SEQ ID NO: 58: 5'- gtg ggt tga gaa tgc ttt tga tgt ggc c - 3'

SEQ ID NO: 59: 5'- gag ttc gtg ctg.tcc aac ttt ggc cac atc - 3'

SEQ ID NO: 60: 5'- gat gtg gcc aaa gtt gga cag cac gaa ctc - 3'

SEQ ID NO: 61: 5'- ttc acc cag atg gtc acc gtg ctc gac ttc aac - 3'

SEQ ID NO: 62: 5'- gtt gaa gtc gag cac ggt gac cat ctg ggt gaa - 3'

SEQ ID NO: 63: 5'- gag ttc gtg ctg tcc aac ggc ggc cac atc - 3'

SEQ ID NO: 64: 5'- gat gtg gcc gcc gtt gga cag cac gaa ctc - 3'

SEQ ID NO: 65: 5'- atc aac ctg ctg acc gat gcg atg tcg ccg acc - 3'

SEQ ID NO: 66: 5'- ggt egg cga cat cgc atc ggt cag cag gtt gat - 3'

SEQ ID NO: 67: 5'- ggt cac atc aaa agc atc ctc aac ccac - 3'

SEQ ID NO: 68: 5'- gtg ggt tga gga tgc ttt tga tgt gac c - 3'

SEQ ID NO: 69: 5'- gag ttc atc ctc tcc aac ttt ggt cac atc - 3'

SEQ ID NO: 70: 5'- gat gtg acc aaa gtt gga gag gat gaa ctc - 3'

SEQ ID NO: 71: 5'- ttc acc caa ctg gtc acc gtg ctc gac ttc gaa - 3'

SEQ ID NO: 72: 5'- ttc gaa gtc gag cac ggt gac cag ttg ggt gaa - 3'

SEQ ID NO: 73: 5'- gag ttc atc ctc tcc aac ggc ggt cac atc - 3'

SEQ ID NO: 74: 5'- gat gtg acc gcc gtt gga gag gat gaa ctc - 3'

The mutants of the synthase prepared from polyhydroxyalkanoate synthase derived from *Pseudomonas* sp. MBEL 6-19, PhaC1_{*Ps6-19*,} and four Type II polyhydroxyalkanoate synthases prepared according to the present invention were introduced into the constitutive expression system in an operon type that is expressed along with the mutant gene (*pct540_{Cp}*; WO09/022797) of propionyl-CoA transferase, a monomer supply enzyme. In order to confirm that the prepared synthase mutants exhibit the activity of synthesis of a lactate copolymer [P(3HB-co-LA)], the recombinant vectors as listed in Table 3 were transformed to *E. coli* XL-1 Blue, and then grown in a P(3HB-co-LA) detection medium (LB agar, glucose 20 g/L, 3HB 2' g/L, Nile red 0.5 µg/mL). As a result, it was found that the lactate copolymer was produced in all of *E. coli* XL-1 Blue transformed with the recombinant vectors comprising the mutants of synthases with the amino acid mutations, even though it did differ slightly depending on the mutants, and the lactate copolymer was not produced in *E. coli* XL-1 Blue that expressed wild-type synthase without introducing the amino acid mutation. That is, as mentioned in Example 1, it was found that all of PhaC1*_{Pch},* PhaC1*_{Ppu}*, PhaC1*_{Pre},* and PhaC1*_{Pae}* used for the present invention in addition to PhaC1*_{Ps6-19}* had newly formed or increased activities of production of a lactate copolymer due to the amino acid mutation in the positions of Glu130, Ser325, Ser477, and Gln481.

**<Example 3> Production of Lactate Polymer and Copolymer Using Synthase Mutant**

In order to quantitatively analyze the activities of synthesis of five wild-type Type II polyhydroxyalkanoate synthases (PhaC1*_{Ps6-19},* PhaC1*_{Pch}*, PhaC1*_{Ppu}*, PhaC1*_{Pre}*, PhaC1*_{Pae}*) prepared in Example 2 and lactate copolymer of mutants thereof [P(3HB-co-LA)], *E. coli* XL-1 Blue transformed with the recombinant expression vectors (Table 3) comprising the above-mentioned synthases was cultured in a flask containing MR medium supplemented with glucose (20 g/L) and 3HB (2 g/L) at 30 °C for 4 days. Compositions of MR medium used for the present invention were as listed in Table 4. The cultured bacteria was collected through centrifugation, washed three times with distilled water, and then dried in an oven at 100 °C for 24 hours. The compositions and content of the polymer synthesized in the dried cell were analyzed through gas chromatography, and the results are shown in Table 5.

**[Table 4]**

| Composition of MR medium used for Culture of Recombinant *E. coli* | |
|---|---|
| Components | Modified R (MR) (/L) |
| KH₂PO₄ | 6.67 g |
| (NH₄)₂HPO₄ | 4 g |
| Citrate | 0.8 g |
| MgSO₄.H₂O | 0.8 g |
| Micro ingredient | 5 mL |

^{*}Micro Ingredient (/L): FeSO₄ • H₂O, 10 g; ZnSO₄ • H₂O, 2.25 g; CuSO₄ • H₂O, 1 g; MnSO₄ • H₂O, 0.5 g; CaCl₂ • H₂O, 2 g; Na₂B₄O₇ • H₂O, 0.23 g; (NH₄)₆Mo₇O₂₄, 0.1 g; 35 % HC1, 10 mL.

**[Table 5]**

| Synthesis of Lactate Copolymer Using Synthase Mutant | | | | |
|---|---|---|---|---|
| Source | PHA Synthase | Amino acid substitution | Content of Polymer (wt %) | LA Fragment |
| *Pseudomonas* sp. MBEL 6-19 | PhaC1*_{Ps6-19}* | - | 0.6± 0.1 | 0 |
| | PhaC1202*_{Ps6-19}* | E130D, Q481K | 39.8± 5. 1 | 38.9± 1. 7 |
| | PhaC1301*_{Ps-19}* | E130D, S477F, Q481K | 49.9± 8.2 2 | 36.2± 3.3 |
| | PhaC1310*_{Ps6-19}* | E130D, S325T, Q481K | 50.9± 2.7 | 43.9± 1.8 |
| | *FhaC1339_{Ps-19}* | *E130D, S325T, S477F, Q481K* | *59.4± 1. 6* | *45.4± 3.6* |
| | *PhaC1337_{Ps-19}* | E130D, S325T, S477G Q481K | 55.4± 3.5 | 51.7± 2.6 |
| *Pseudomonas chlororaphis* | ThaC1*_{Pch}* | - | 0.2± 0 | 0 |
| | PhaC1202*_{Pch}* | E130D, Q481K | 13.2± 3. 4 | 20.1± 0. 4 |
| | *PhaC1301_{Pch}* | E130D, S477F, Q481K | 21.3± 3. 5 | 18.8± 1. 2 |
| | *PhaC1310_{Pch}* | E130D, S325T, Q481K | 27.5± 4.4 | 23.2± 7.6 |
| | *PhaC1339_{Pch}* | *E130D, S325T, S477F, Q481K* | *46.8± 3.1* | *34.6± 2.0* |
| | *PhaC1337_{Pch}* | E130D, S325T, S477G, Q481K | 52.8± 1.4 | 43.7± 1.8 |
| *Pseudomonas putida* KT2440 | PhaC1*_{Ppu}* | - | 0 | 0 |
| | *PhaC1202_{Ppu}* | E130D, Q481K | 6.8± 0.9 | 0 |
| | PhaC1301*_{Ppu}* | E130D, S477F, Q481K | 7.8± 0.5 | 7.1± 0.8 |
| | PhaC1310*_{Ppu}* | E130D, S325T, Q481K | 39.3± 0.6 | 27.5± 0.8 |
| | PhaC1339*_{Ppu}* | *E130D, S325T, S477F, Q481K* | *41.3± 3.1* | *25.1± 0.3* |
| | *PhaC1337_{Ppu}* | E130D, S325T, S477G, Q481K | 43.0± 2.2 | 32.0±2.6 |
| *Pseudomonas resinovorans* | PhaC1*_{Pre}* | - | 1.4± 0 | 39.8± 4.5 |
| | PhaC1202*_{Pre}* | E130D, Q481K | 31.6± 4.4 | 62.7± 2.1 |
| | PhaC1301*_{Pre}* | E130D, S477F, Q481K | 30.8± 2.9 | 55.3± 2.3 |
| | PhaC1310*_{Pre}* | E130D, S325T Q481K | 57.5± 0.4 | 49.0± 2.4 |
| | PhaC1339*_{Pre}* | *E130D, S325T, S477F, Q481K* | *52.1± 0.4* | *56.5± 0.3* |
| | *PhaC1337_{Pre}* | E130D, S325T, S477G, Q481K | 53.6± 2.9 | 65.5± 1.0 |
| *Pseudomonas aeruginosa* PAO1 | PhaC1*_{Pae}* | - | 0 | 0 |
| | PhaC1202*_{Pae}* | E130D, Q481K | 5.1± 1.1 | 30.7± 3. 1 |
| | PhaC1301*_{Pae}* | E130D, S477F, Q481K | 8.4±0.6 | 21.5±0.5 |
| | PhaC1310*_{Pae}* | E130D, S325T, Q481K | 36.0± 1.3 | 36.2± 0.1 |
| | PhaC1339*_{Pae}* | *E130D, S325T, S477F, G481K* | *39.9± 1.1* | *40.1± 0.6* |
| | *PhaC1337_{Pae}* | E130D, S325T S477G, Q481K | 49.2± 2.9 | 52.7± 0.5 |

As a result of gas chromatography analysis, while lactate copolymer was not produced or produced in a very small amount (1.4 wt%) in the recombinant *E. coli* XL-1 Blue that expressed five wild-type Type II polyhydroxyalkanoate synthases (PhaC1*_{Ps6-19}*, PhaC1*_{Pch},* PhaC1*_{Ppu},* PhaC1*_{Pre}*, PhaC1*_{Pae}*), a lactate copolymer [P(3HB-co-LA)] was accumulated in a cell because of the activity that can accept lactyl-CoA as a substrate was newly generated (or increased) in the case of the mutants with the amino acid mutations in the positions of Gau130, Ser325, Ser477, and Glen481. For the compositions and content of the synthesized lactate copolymer in a cell, the mutants introduced with the mutations of E130D, S325T, S477G, and Q481 K at the same time exhibited a high accumulation rate of a polymer in a cell and high synthesis activity of a copolymer, thereby showing high content of lactate, depending on a type of each polyhydroxyalkanoate synthase. Also, the synthesis activity of a lactate polymer of the mutants introduced with E130D and Q481K and the mutants introduced with E130D, S325T, S477G, and Q481K at the same time was analyzed among the mutants as listed in Table 3. To achieve this, *E. coli* XL-1 Blue that expressed each mutation was cultured in a flask comprising MR medium supplemented with glucose (20 g/L) at 30 °C for 4 days. As an analysis result, the mutants with all of E130D, S325T, S477G, and Q481K produced about 2 to 7 wt% lactate polymer when it was used to synthesize the lactate polymer, and the activity of the mutant of synthase derived from *P. resinovorans* was the highest *(see* Table 6).

**[Table 6]**

| Synthesis of Lactate Polymer using Synthase Mutant | | | |
|---|---|---|---|
| Source | PHA Synthase | Amino acid substitution | PLA (wt%) |
| *Pseudomonas* sp. MBEL6-19 | PhaC1202_{*Ps6-1*9} | E130D, Q481K | 0.5± 0.1 |
| | PhaC1337*_{Ps6-19}* | E130D, S325T, S477G, Q481K | 7.2±0.3 |
| *Pseudomonas chlororaphis* | PhaC1202*_{Pch}* | E130D, Q481K | 00.3± |
| | PhaC1337*_{Pch}* | E130D, S325T, S477G, Q481K | 4.5± 0.4 |
| *Pseudomonas putida* KT2440 | *PhaC1202_{Ppu}* | E130D, Q481K | 0 |
| | PhaC1337*_{Ppu}* | E130D, S325T, S477G, Q481K | 2.0± 0.1 |
| *Pseudomonas resinovorans* | PhaC1202*_{Pre}* | E130D, Q481K | 1.2± 0.3 |
| | PhaC1337*_{Pre}* | E130D, S325T, S477G, Q481K | 7.3± 0 |
| *Pseudomonas aeruginosa* PAO1 | *PhaC1202_{Pae}* | E130D, Q481K | 0.6± 0.3 |
| | *PhaC1337_{Pae}* | E130D, S325T, S477G, Q481K | 5.9± 0.4 |

All of the polyhydroxyalkanoate synthases according to the present invention can have an activity of synthesizing a lactate polymer and a lactate copolymer by an amino acid sequence mutation affecting the activity of synthesizing a lactate polymer and a lactate copolymer, and then can produce a lactate copolymer having different features, respectively, when using the mutants of synthases.

While the invention has been shown and described with reference to certain exemplary embodiments thereof, it will be understood by those skilled in the art that various changes in form and details may be made therein without departing from the spirit and scope of the invention as defined by the appended claims.

## Claims

1. A mutant of polyhydroxyalkanoate synthase for synthesizing a lactate polymer or a lactate copolymer using lactyl-CoA as a substrate, in which the mutant has at least one of mutation selected from the group consisting of
a) E130D and Q481K;
b) E130D, S325T, and Q481K;
c) E130D, S477F, and Q481K;
d) E130D, S325T, S477F, and Q481K; and
e) E130D, S325T, S477G and Q481K;
in an amino acid sequence of polyhydroxyalkanoate synthase set forth in SEQ ID NO: 2, 4, 6 or 8.

2. A gene encoding the mutant of polyhydroxyalkanoate synthase of claim 1.

3. A recombinant vector for synthesizing a lactate polymer or a lactate copolymer comprising the gene of claim 2.

4. The recombinant vector of claim 3, further comprising a propionyl-CoA transferase gene (pct) of SEQ ID NO: 77.

5. The recombinant vector of claim 4, wherein the propionyl-CoA transferase gene of SEQ ID NO: 77 includes the propionyl CoA-transferase mutant gene comprising the mutation selected from the group consisting of
a) A1200G mutation in a base sequence of SEQ ID NO: 77;
b) T78C, T669C, A1125G, and T1158C mutations in a base sequence of SEQ ID NO: 77;
c) Gly335Asp mutation in an amino acid sequence of SEQ ID NO: 78 and A1200G mutations in a base sequence of SEQ ID NO: 77;
d) Ala243Thr mutation in an amino acid sequence of SEQ ID NO: 78 and A1200G mutation in a base sequence of SEQ ID NO: 77;
e) Asp65Gly mutation in an amino acid sequence of SEQ ID NO: 78 and T669C, A1125G, and T1158C mutations in a base sequence of SEQ ID NO: 77;
f) Asp257Asn mutation in an amino acid sequence of SEQ ID NO: 78 and A1200G mutation in a base sequence of SEQ ID NO: 77;
g) Asp65Asn mutation in an amino acid sequence of SEQ ID NO: 78 and T699C, A1125G, and T1158C mutations in a base sequence of SEQ ID NO: 77;
h) Thr199Ile mutation in an amino acid sequence of SEQ ID NO: 78 and T699C, A1125G, and T1159C mutations in a base sequence of SEQ ID NO: 77; and
i) Va1193Ala mutation in an amino acid sequence of SEQ ID NO: 78 and T78C, T699C, and T1158C mutations in a base sequence of SEQ ID NO: 77.

6. A transformant transformed with the recombinant vector any one of claims 3 to 5.

7. A method of preparing a lactate polymer or a lactate copolymer, comprising culturing the transformant of claim 6.

8. The method of claim 7, wherein the culturing is performed in an environment containing hydroxyalkanoate and the copolymer is hydroxyalkanoate-co-3-lactate.

9. The method of claim 8, wherein the hydroxyalkanoate is at least one selected from the group consisting of 3-hydroxybutyrate, 3-hydroxyvalerate, 4-hydroxybutyrate, (D)-3-hydroxycarboxylic acids with a medium chain length of 6 to 14 carbon numbers, 3-hydroxypropionic acid, 3-hydroxyhexanic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytetradecanoic acid, 3-hydroxyhexadecanoic acid, 4-hydroxyvaleric acid, 4-hydroxyhexanoic acid, 4-hydroxyheptanoic acid, 4-hydroxyoctanoic acid, 4-hydroxydecanoic acid, 5-hydroxyvaleric acid, 5-hydroxyhexanoic acid, 6-hydroxydodecanoic acid, 3-hydroxy-4-pentenoic acid, 3-hydroxy-4-trans-hexenoic acid, 3-hydroxy-4-cis-hexenoic acid, 3-hydroxy-5-hyxenoic acid, 3-hydroxy-6-trans-octenoic acid, 3-hydroxy-6-cis octenoic acid, 3-hydroxy-7-octenoic acid, 3-hydroxy-8-nonenoic acid, 3-hydroxy-9-decenoic acid, 3-hydroxy-5-cis-dodecenoic acid, 3-hydroxy-5-cis-dodecenoic acid, 3-hydroxy-5-cis-tetradecenoic acid, 3-hydroxy-7-cis-tetradecenoic acid, 3-hydroxy-5,8-cis-cis-tetradecenoic acid, 3-hydroxy-4-methylvaleric acid, 3-hydroxy-4-methylhexanoic acid, 3-hydroxy-5-methylhexanoic acid, 3-hydroxy-6-methylhexanoic acid, 3-hydroxy-4-methyloctanoic acid, 3-hydroxy-5-methyloctanoic acid, 3-hydroxy-6-methyloctanoic acid, 3-hydroxy-7-methyloctanoic acid, 3-hydroxy-6-methylnonanoic acid, 3-hydroxy-7-methylnonanoic acid, 3-hydroxy-8-methylnonanoic acid, 3-hydroxy-7-methyldecanoic acid, 3-hydroxy-9-methyldecanoic acid, 3-hydroxy-7-methyl-6-octenoic acid, malic acid, 3-hydroxysuccinic acid-methylester, 3-hydroxyadipinic acid-methylester, 3-hydroxysuberic acid-methylester, 3-hydroxyazelaic acid-methylester, 3-hydroxysebacic acid-methylester, 3-hydroxysuberic acid-ethylester, 3-hydroxysebacic acid-ethylester, 3-hydroxypimelic acid-propyl ester, 3-hydroxysebacic acid-benzylester, 3-hydroxy-8-acetoxyoctanoic acid, 3-hydroxy-9-acetoxynonanoic acid, phenoxy-3-hydroxybutyric acid, phenoxy-3-hydroxyvaleric acid, phenoxy-3-hydroxyheptanoic acid, phenoxy-3-hydroxyoctanoic acid, para-cyanophenoxy-3-hydroxybutyric acid, para-cyanophenoxy-3-hydroxyvaleric acid, para-cyanophenoxy-3-hydroxyhexanoic acid, para-nitrophenoxy-3-hydroxyhexanoic acid, 3-hydroxy-5-phenylvaleric acid, 3-hydroxy-5-cyclohexylbutyric acid, 3,12-dihydroxydodecanoic acid, 3,8-dihydroxy-5-cis-tetradecenoic acid, 3-hydroxy-4,5-epoxydecanoic acid, 3-hydroxy-6,7-epoxydodecanoic acid, 3-hydroxy-8,9-epoxy-5,6-cis-tetradecanoic acid, 7-cyano-3-hydroxyheptanoic acid, 7-cyano-3-hydroxyheptanoic acid, 3-hydroxy-7-fluoroheptanoic acid, 3-hydroxy-9-fluorononanoic acid, 3-hydroxy-6-chlorohexanoic acid, 3-hydroxy-8-chlorooctanoic acid, 3-hydroxy-6-bromohexanoic acid, 3-hydroxy-8-bromooctanoic acid, 3-hydroxy-11-bromoundecanoic acid, 3-hydroxy-2-butenoic acid, 6-hydroxy-3-dodecenoic acid, 3-hydroxy-2-methylbutyric acid, 3-hydroxy-2-methylvaleric acid and 3-hydroxy-2,6-dimethyl-5-heptenoic acid.


<110>    LG CHEMICAL LTD.

<120>    Mutants of PHA Synthases and Method for Preparing PLA and PLA
         Copolymer Using the Same

<130>    X100029

<150>    KR10-2009-0059488
<151>    2009-06-30

<160>    78

<170>    KopatentIn 1.71

<210>    1
<211>    1677
<212>    DNA
<213>    PHA synthase from Pseudomonas chlororaphis KCTC 12349 (phaC1Pch)

<400>    1
atgagtaaca agaataacga tgacctgaaa cgccaagcct cggaaaacac tctgggcctg      60

aatcctgtca tcggtttacg tcggaaagat ctgcttacct ctgctcgtat ggtgctgacc     120

caggccatca aacaaccgct gcacagcgcc aaacatgtgg cccatttcgg cgtcgaactg     180

aagaacgtga tgttcggcaa atcgacgctg caaccggaga gcgatgaccg tcgcttcaac     240

gatccggcct ggagccagaa cccgctgtac aagcgttacc tgcaaaccta cctggcgtgg     300

cgcaaggagc ttcacgactg gatcggccac agcaacctct ccgaacagga catcagtcgc     360

gcccacttcg tcatcaacct gatgaccgaa gccatggcgc cgaccaacac cgccgccaac     420

cccgcggcgg tcaaacgctt cttcgagacc ggcggcaaaa gcctgctcga tggcctctcc     480

catctggcca aggacctggt gcacaacggc ggcatgccga gccaggtcaa catggacgcc     540

ttcgagatcg gcaagaacct gggcaccagt gaaggcgcgg tggtatttcg caacgacgtg     600

ctggaactga tccagtaccg cccggtgacc gagcaggtgc acgaacgccc gctgctggtg     660

gtcccgccac agatcaacaa gttctacgtc ttcgacctca gcccggacaa gagcctggcg     720

cgtttctgcc tgcgcaacgg cgtgcagacc tttatcgtca gttggcgcaa cccgaccaag     780

gcgcagcgcg agtggggcct gtcgacctac atcgaagcgc tgaaagaagc ggtcgacgtg     840

gtcaccgcca tcaccggcag caaggacgtg aacatgctcg gcgcctgctc cggtggcatc     900

acctgcaccg cgctgctggg gcactacgcg gccctgggcg agaagaaggt caacgccctg     960

accctgctgg tcagcgtgct cgataccacc ctcgacaccc aggtggcgct gttcgtcgac    1020

gagcagaccc tggaagccgc caagcgtcat tcctatcagg ctggcgtgct ggaaggccgc    1080

gacatggcca aggtcttcgc ctggatgcgg cccaacgacc tgatctggaa ctactgggtg    1140

aacaactacc tgctgggcaa cgagccgccg gtgttcgaca tcctgttctg gaacaacgac    1200

accacccggt tgcccgcggc cttccacggc gacctgatcg aaatgttcaa aaacaatcca    1260

ctgatccgcc ccaacgcact ggaagtgtgc ggtacgccga tcgacctgaa acaggtcacc    1320

gccgacatct tctctctggc cggcaccaac gatcacatca ccccatggaa gtcctgctac    1380

aagtcggccc aactgttcgg cggcaaggtc gaattcgtcc tctccagcag cgggcatatc 1440

cagagcatcc tcaacccgcc gggcaacccc aagtcgcgct acatgaccag cgaagagatg 1500

ccgcccagcg ccgacgactg gcaggaaaac tccaccaagc acactgactc ctggtggctg 1560

cactggcagg cctggcaggc ggagcgctcg ggcaaactga aaaagacgcc gagcattctc 1620

ggcaacaagg cgtatccagc ggccgaggcg gcgccgggta cttatgtcca tgaacgt 1677

<210> 2
<211> 559
<212> PRT
<213> PHA synthase from Pseudomonas chlororaphis KCTC 12349 (PhaC1Pch)

<400> 2
Met Ser Asn Lys Asn Asn Asp Asp Leu Lys Arg Gln Ala Ser Glu Asn
1               5                   10                  15

Thr Leu Gly Leu Asn Pro Val Ile Gly Leu Arg Arg Lys Asp Leu Leu
            20                  25                  30

Thr Ser Ala Arg Met Val Leu Thr Gln Ala Ile Lys Gln Pro Leu His
        35                  40                  45

Ser Ala Lys His Val Ala His Phe Gly Val Glu Leu Lys Asn Val Met
    50                  55                  60

Phe Gly Lys Ser Thr Leu Gln Pro Glu Ser Asp Asp Arg Arg Phe Asn
65                  70                  75                  80

Asp Pro Ala Trp Ser Gln Asn Pro Leu Tyr Lys Arg Tyr Leu Gln Thr
                85                  90                  95

Tyr Leu Ala Trp Arg Lys Glu Leu His Asp Trp Ile Gly His Ser Asn
            100                 105                 110

Leu Ser Glu Gln Asp Ile Ser Arg Ala His Phe Val Ile Asn Leu Met
        115                 120                 125

Thr Glu Ala Met Ala Pro Thr Asn Thr Ala Ala Asn Pro Ala Ala Val
    130                 135                 140

Lys Arg Phe Phe Glu Thr Gly Gly Lys Ser Leu Leu Asp Gly Leu Ser
145                 150                 155                 160

His Leu Ala Lys Asp Leu Val His Asn Gly Gly Met Pro Ser Gln Val
                165                 170                 175

Asn Met Asp Ala Phe Glu Ile Gly Lys Asn Leu Gly Thr Ser Glu Gly
            180                 185                 190

Ala Val Val Phe Arg Asn Asp Val Leu Glu Leu Ile Gln Tyr Arg Pro
        195                 200                 205

Val Thr Glu Gln Val His Glu Arg Pro Leu Leu Val Val Pro Pro Gln
    210                 215                 220

Ile Asn Lys Phe Tyr Val Phe Asp Leu Ser Pro Asp Lys Ser Leu Ala
225                 230                 235                 240

Arg Phe Cys Leu Arg Asn Gly Val Gln Thr Phe Ile Val Ser Trp Arg
                245                 250                 255

Asn Pro Thr Lys Ala Gln Arg Glu Trp Gly Leu Ser Thr Tyr Ile Glu
            260                 265                 270

Ala Leu Lys Glu Ala Val Asp Val Val Thr Ala Ile Thr Gly Ser Lys
        275                 280                 285

Asp Val Asn Met Leu Gly Ala Cys Ser Gly Gly Ile Thr Cys Thr Ala
    290                 295                 300

Leu Leu Gly His Tyr Ala Ala Leu Gly Glu Lys Lys Val Asn Ala Leu
305                 310                 315                 320

Thr Leu Leu Val Ser Val Leu Asp Thr Thr Leu Asp Thr Gln Val Ala
                325                 330                 335

Leu Phe Val Asp Glu Gln Thr Leu Glu Ala Ala Lys Arg His Ser Tyr
            340                 345                 350

Gln Ala Gly Val Leu Glu Gly Arg Asp Met Ala Lys Val Phe Ala Trp
        355                 360                 365

Met Arg Pro Asn Asp Leu Ile Trp Asn Tyr Trp Val Asn Asn Tyr Leu
    370                 375                 380

Leu Gly Asn Glu Pro Pro Val Phe Asp Ile Leu Phe Trp Asn Asn Asp
385                 390                 395                 400

Thr Thr Arg Leu Pro Ala Ala Phe His Gly Asp Leu Ile Glu Met Phe
                405                 410                 415

Lys Asn Asn Pro Leu Ile Arg Pro Asn Ala Leu Glu Val Cys Gly Thr
            420                 425                 430

Pro Ile Asp Leu Lys Gln Val Thr Ala Asp Ile Phe Ser Leu Ala Gly
        435                 440                 445

Thr Asn Asp His Ile Thr Pro Trp Lys Ser Cys Tyr Lys Ser Ala Gln
    450                 455                 460

Leu Phe Gly Gly Lys Val Glu Phe Val Leu Ser Ser Ser Gly His Ile
465                 470                 475                 480

Gln Ser Ile Leu Asn Pro Pro Gly Asn Pro Lys Ser Arg Tyr Met Thr
                485                 490                 495

Ser Glu Glu Met Pro Pro Ser Ala Asp Asp Trp Gln Glu Asn Ser Thr
            500                 505                 510

Lys His Thr Asp Ser Trp Trp Leu His Trp Gln Ala Trp Gln Ala Glu
        515                 520                 525

Arg Ser Gly Lys Leu Lys Lys Thr Pro Ser Ile Leu Gly Asn Lys Ala
    530                 535                 540

Tyr Pro Ala Ala Glu Ala Ala Pro Gly Thr Tyr Val His Glu Arg
545                 550                 555


<210>    3
<211>    1677
<212>    DNA
<213>    PHA synthase from Pseudomonas putida KT2440 ATCC 47054 (phaC1Ppu)

<400>    3

atgagtaaca agaacaacga tgagctacag cggcaggcct cggaaaacac cctggggctg 60

aacccggtca tcggcatccg ccgcaaggac ctgttgagct cggcacgcac cgtgctgcgc 120

caggccgtgc gccaaccgct gcacagcgcc aagcatgtgg ctcactttgg cctggagctg 180

aagaacgtgt tgctgggcaa atccagcctg gccccggaca gcgacgaccg tcgcttcaat 240

gacccggcct ggagcaacaa cccgctgtac cgccgctacc tgcaaaccta cctggcctgg 300

cgcaaggagc tgcaggactg ggtgagcagc agcgacctgt ccccccagga catcagccgc 360

ggccagttcg tcatcaacct gatgaccgag gccatggcgc cgaccaatac cctgtccaac 420

ccggctgcgg tcaaacgctt cttcgaaacc ggcggcaaga gcctgctcga tggcctgtcc 480

aacctggcca aggacatggt caacaacggc ggcatgccca gccaggtgaa catggatgcc 540

ttcgaagtgg gcaagaacct gggcaccagc gaaggcgcgg tggtgtaccg caacgatgtg 600

ctggaactga tccagtacag ccccatcacc gagcaggtgc atgcccgtcc gctgctggtg 660

gtgccaccgc agatcaacaa gttctacgtg ttcgacctca gcccggaaaa gagcctggcg 720

cgcttctgcc tgcgctcgca gcagcagacc ttcatcatca gctggcgcaa cccgaccaag 780

gcccagcgtg aatggggcct gtccacctac atcgatgcgc tgaaagaagc cgtcgacgcg 840

gtgctgtcga ttaccggcag caaggacctg aacatgctcg gcgcctgctc cggtggcatc 900

acttgtaccg cactggtggg ccactatgcc gccattggcg agaacaaggt caacgccctg 960

accctgctgg tcagcgtgct ggacaccacc atggacaacc aggttgcttt gtttgtcgac 1020

gagcagacct tggaggccgc caagcgccac tcctatcagg cgggcgtgct ggaaggcagc 1080

gaaatggcca aggtgttcgc ctggatgcgc cccaacgacc tgatctggaa ctactgggta 1140

aacaactacc tgctcggcaa tgagcccccc gtgttcgaca tcctgttctg gaacaacgac 1200

accacgcgcc tgccggccgc cttccacggc gacctgatcg aaatgttcaa gagcaacccg 1260

ctgacccgcc ccgacgccct ggaagtgtgc ggcaccgcga tcgacctgaa acaggtcaaa 1320

tgcgacatct acagcctcgc cggcaccaac gaccacatca ccccctggcc gtcatgctac 1380

cgctcggcac atctgttcgg cggcaagatc gaattcgtac tgtccaacag cgggcatatc 1440

cagagcatcc tcaacccgcc gggcaacccg aaggcacgtt tcatgaccgg tgccgatcgc 1500

ccgggtgacc cggtggcctg gcaggaaaat gccatcaagc atgcagactc ctggtggttg 1560

cactggcaga gttggctggg cgagcgtgcc ggcgcgctga aaaaggcacc gacccgcctg 1620

ggcaaccgta cctatgccgc cggcgaagcc tccccaggca cctacgttca cgagcgt 1677

<210> 4
<211> 559
<212> PRT
<213> PHA synthase from Pseudomonas putida KT2440 ATCC 47054 (PhaC1Ppu)

<400> 4
Met Ser Asn Lys Asn Asn Asp Glu Leu Gln Arg Gln Ala Ser Glu Asn
1               5                   10                  15

Thr Leu Gly Leu Asn Pro Val Ile Gly Ile Arg Arg Lys Asp Leu Leu
            20                  25                  30

Ser Ser Ala Arg Thr Val Leu Arg Gln Ala Val Arg Gln Pro Leu His
        35                  40                  45

Ser Ala Lys His Val Ala His Phe Gly Leu Glu Leu Lys Asn Val Leu
    50                  55                  60

Leu Gly Lys Ser Ser Leu Ala Pro Asp Ser Asp Asp Arg Arg Phe Asn
65                  70                  75                  80

Asp Pro Ala Trp Ser Asn Asn Pro Leu Tyr Arg Arg Tyr Leu Gln Thr
                85                  90                  95

Tyr Leu Ala Trp Arg Lys Glu Leu Gln Asp Trp Val Ser Ser Ser Asp
            100                 105                 110

Leu Ser Pro Gln Asp Ile Ser Arg Gly Gln Phe Val Ile Asn Leu Met
        115                 120                 125

Thr Glu Ala Met Ala Pro Thr Asn Thr Leu Ser Asn Pro Ala Ala Val
    130                 135                 140

Lys Arg Phe Phe Glu Thr Gly Gly Lys Ser Leu Leu Asp Gly Leu Ser
145                 150                 155                 160

Asn Leu Ala Lys Asp Met Val Asn Asn Gly Gly Met Pro Ser Gln Val
                165                 170                 175

Asn Met Asp Ala Phe Glu Val Gly Lys Asn Leu Gly Thr Ser Glu Gly
            180                 185                 190

Ala Val Val Tyr Arg Asn Asp Val Leu Glu Leu Ile Gln Tyr Ser Pro
        195                 200                 205

Ile Thr Glu Gln Val His Ala Arg Pro Leu Leu Val Val Pro Pro Gln
    210                 215                 220

Ile Asn Lys Phe Tyr Val Phe Asp Leu Ser Pro Glu Lys Ser Leu Ala
225                 230                 235                 240

Arg Phe Cys Leu Arg Ser Gln Gln Gln Thr Phe Ile Ile Ser Trp Arg
                245                 250                 255

Asn Pro Thr Lys Ala Gln Arg Glu Trp Gly Leu Ser Thr Tyr Ile Asp
            260                 265                 270

Ala Leu Lys Glu Ala Val Asp Ala Val Leu Ser Ile Thr Gly Ser Lys
        275                 280                 285

Asp Leu Asn Met Leu Gly Ala Cys Ser Gly Gly Ile Thr Cys Thr Ala
    290                 295                 300

Leu Val Gly His Tyr Ala Ala Ile Gly Glu Asn Lys Val Asn Ala Leu
305                 310                 315                 320

Thr Leu Leu Val Ser Val Leu Asp Thr Thr Met Asp Asn Gln Val Ala
                325                 330                 335

Leu Phe Val Asp Glu Gln Thr Leu Glu Ala Ala Lys Arg His Ser Tyr
            340                 345                 350

Gln Ala Gly Val Leu Glu Gly Ser Glu Met Ala Lys Val Phe Ala Trp

355                 360                 365

Met Arg Pro Asn Asp Leu Ile Trp Asn Tyr Trp Val Asn Asn Tyr Leu
    370                 375                 380

Leu Gly Asn Glu Pro Pro Val Phe Asp Ile Leu Phe Trp Asn Asn Asp
385                 390                 395                 400

Thr Thr Arg Leu Pro Ala Ala Phe His Gly Asp Leu Ile Glu Met Phe
                405                 410                 415

Lys Ser Asn Pro Leu Thr Arg Pro Asp Ala Leu Glu Val Cys Gly Thr
            420                 425                 430

Ala Ile Asp Leu Lys Gln Val Lys Cys Asp Ile Tyr Ser Leu Ala Gly
        435                 440                 445

Thr Asn Asp His Ile Thr Pro Trp Pro Ser Cys Tyr Arg Ser Ala His
    450                 455                 460

Leu Phe Gly Gly Lys Ile Glu Phe Val Leu Ser Asn Ser Gly His Ile
465                 470                 475                 480

Gln Ser Ile Leu Asn Pro Pro Gly Asn Pro Lys Ala Arg Phe Met Thr
                485                 490                 495

Gly Ala Asp Arg Pro Gly Asp Pro Val Ala Trp Gln Glu Asn Ala Ile
            500                 505                 510

Lys His Ala Asp Ser Trp Trp Leu His Trp Gln Ser Trp Leu Gly Glu
        515                 520                 525

Arg Ala Gly Ala Leu Lys Lys Ala Pro Thr Arg Leu Gly Asn Arg Thr
    530                 535                 540

Tyr Ala Ala Gly Glu Ala Ser Pro Gly Thr Tyr Val His Glu Arg
545                 550                 555

<210>    5
<211>    1677
<212>    DNA
<213>    PHA synthase from Pseudomonas resinovorans KCTC 12498 (phaC1Pre)

<400>    5
atgagcaaca agaacaatga agacctgcag cgccaggcct cggacaacac cctgaacctc      60

aatccggtga taggtatccg cggcaaggat ctgctgtctt cagcacggat ggtgctgttg     120

caagccatca agcaaccttt ccacagtgca aaacacgtcg cccatttcgg cctggaattg     180

aaaaacgtgt tgcttggcca gtccggtctg caaccggaag ccgatgaccg tcgcttcaat     240

gatccggcct ggagccagaa cccgctgtac aagcgctatc tgcagaccta cctcgcctgg     300

cgcaaggagc tgcacagctg gatcgacgag agcaacctct cgtcacagga tgccagtcgc     360

ggccacttcg tcatcaacct gatgaccgag gcgatggcgc ccaccaacag catggccaac     420

ccggccgcag tcaaacggtt cttcgagacc ggcggcaaga gcctgctgga tggcctctcc     480

cacctggcca aggacatggt gaacaacggc ggcatgccga gccaggtcaa catggatgcc     540

ttcgaggtcg gccagaacct cgccacgacc gaaggtgcgg tggttttccg caatgacgtg     600

ctggaactga tccagtacaa gcccatcacc gagagcgtgt acgaacgccc gctactcgtg 660

gtgccgcccc agatcaacaa gttctacgtc ttcgacctgt caccggagaa gagcctcgcg 720

cgcttctgcc tgcgcagcaa cctgcagacc ttcatcgtca gctggcgcaa cccgaccaag 780

gcgcagcgcg agtggggcct gtccacgtac atcgaggccc tcaaggaggc cattgacgtc 840

atcctgaaga tcaccggcgc caaggacctc aacatcctcg gcgcgtgctc cggcggcatc 900

accacggtcg ccctgctcgg ccactaccag gccattggtg aaaccaaggt caatgccttc 960

acccagatgg tcagcgtgct cgacttcaac ctcgatagcc aggtcgcgct gttcgccgac 1020

gagcagaccc tcgaagccgc caagcgccgc tcgtaccagg ccggcgtact ggaaggcaaa 1080

gacatggcca aggtcttcgc ctggatgcgc cccaacgacc tgatctggaa ctactgggtc 1140

aacaactacc tgctgggcaa cgaaccgccg gccttcgaca tcctctattg gaacaacgac 1200

accacgcgtc tgcctgcagc gttccatggc gaactggtgg aaatgttcaa gaccaatgcc 1260

ctgacccgtc caaatgccct ggaggtctgt ggcacgccca tcgacctgaa gcaagtcacc 1320

agcgacttct tctgccttgc cggcaccacc gaccacatta ccccctggga agcctgctac 1380

cgctcggcgc tgctgctggg cggcaaatgc gagttcgtgc tgtccaacag cggccacatc 1440

cagagcattc tcaacccacc gggcaacccc aaggcacgct tctccaccgg cagcgagatg 1500

ccgaaagatc cgaaggcctg gctggaaaat gcgaccaagc acgccgactc ctggtggctg 1560

cattggcagc agtggatcgg cgaacgctcc ggcaagacca agaaagccag ctttaccctg 1620

ggcaacaagg ccttcccggc cggtgaagcc tcgcccggca cctatgtgca cgagcga 1677

<210> 6
<211> 559
<212> PRT
<213> PHA synthase from Pseudomonas resinovorans KCTC 12498 (PhaC1Pre)

<400> 6
Met Ser Asn Lys Asn Asn Glu Asp Leu Gln Arg Gln Ala Ser Asp Asn
1               5                   10                  15

Thr Leu Asn Leu Asn Pro Val Ile Gly Ile Arg Gly Lys Asp Leu Leu
            20                  25                  30

Ser Ser Ala Arg Met Val Leu Leu Gln Ala Ile Lys Gln Pro Phe His
        35                  40                  45

Ser Ala Lys His Val Ala His Phe Gly Leu Glu Leu Lys Asn Val Leu
    50                  55                  60

Leu Gly Gln Ser Gly Leu Gln Pro Glu Ala Asp Asp Arg Arg Phe Asn
65                  70                  75                  80

Asp Pro Ala Trp Ser Gln Asn Pro Leu Tyr Lys Arg Tyr Leu Gln Thr
                85                  90                  95

Tyr Leu Ala Trp Arg Lys Glu Leu His Ser Trp Ile Asp Glu Ser Asn
            100                 105                 110

Leu Ser Ser Gln Asp Ala Ser Arg Gly His Phe Val Ile Asn Leu Met

115                 120                 125

Thr Glu Ala Met Ala Pro Thr Asn Ser Met Ala Asn Pro Ala Ala Val
    130                 135                 140

Lys Arg Phe Phe Glu Thr Gly Gly Lys Ser Leu Leu Asp Gly Leu Ser
145                 150                 155                 160

His Leu Ala Lys Asp Met Val Asn Asn Gly Gly Met Pro Ser Gln Val
                165                 170                 175

Asn Met Asp Ala Phe Glu Val Gly Gln Asn Leu Ala Thr Thr Glu Gly
            180                 185                 190

Ala Val Val Phe Arg Asn Asp Val Leu Glu Leu Ile Gln Tyr Lys Pro
        195                 200                 205

Ile Thr Glu Ser Val Tyr Glu Arg Pro Leu Leu Val Val Pro Pro Gln
    210                 215                 220

Ile Asn Lys Phe Tyr Val Phe Asp Leu Ser Pro Glu Lys Ser Leu Ala
225                 230                 235                 240

Arg Phe Cys Leu Arg Ser Asn Leu Gln Thr Phe Ile Val Ser Trp Arg
                245                 250                 255

Asn Pro Thr Lys Ala Gln Arg Glu Trp Gly Leu Ser Thr Tyr Ile Glu
            260                 265                 270

Ala Leu Lys Glu Ala Ile Asp Val Ile Leu Lys Ile Thr Gly Ala Lys
        275                 280                 285

Asp Leu Asn Ile Leu Gly Ala Cys Ser Gly Gly Ile Thr Thr Val Ala
    290                 295                 300

Leu Leu Gly His Tyr Gln Ala Ile Gly Glu Thr Lys Val Asn Ala Phe
305                 310                 315                 320

Thr Gln Met Val Ser Val Leu Asp Phe Asn Leu Asp Ser Gln Val Ala
                325                 330                 335

Leu Phe Ala Asp Glu Gln Thr Leu Glu Ala Ala Lys Arg Arg Ser Tyr
            340                 345                 350

Gln Ala Gly Val Leu Glu Gly Lys Asp Met Ala Lys Val Phe Ala Trp
        355                 360                 365

Met Arg Pro Asn Asp Leu Ile Trp Asn Tyr Trp Val Asn Asn Tyr Leu
    370                 375                 380

Leu Gly Asn Glu Pro Pro Ala Phe Asp Ile Leu Tyr Trp Asn Asn Asp
385                 390                 395                 400

Thr Thr Arg Leu Pro Ala Ala Phe His Gly Glu Leu Val Glu Met Phe
                405                 410                 415

Lys Thr Asn Ala Leu Thr Arg Pro Asn Ala Leu Glu Val Cys Gly Thr
            420                 425                 430

Pro Ile Asp Leu Lys Gln Val Thr Ser Asp Phe Phe Cys Leu Ala Gly
        435                 440                 445

Thr Thr Asp His Ile Thr Pro Trp Glu Ala Cys Tyr Arg Ser Ala Leu
    450                 455                 460

Leu Leu Gly Gly Lys Cys Glu Phe Val Leu Ser Asn Ser Gly His Ile
465                 470                 475                 480

Gln Ser Ile Leu Asn Pro Pro Gly Asn Pro Lys Ala Arg Phe Ser Thr
                485                 490                 495

Gly Ser Glu Met Pro Lys Asp Pro Lys Ala Trp Leu Glu Asn Ala Thr
            500                 505                 510

Lys His Ala Asp Ser Trp Trp Leu His Trp Gln Gln Trp Ile Gly Glu
        515                 520                 525

Arg Ser Gly Lys Thr Lys Lys Ala Ser Phe Thr Leu Gly Asn Lys Ala
    530                 535                 540

Phe Pro Ala Gly Glu Ala Ser Pro Gly Thr Tyr Val His Glu Arg
545                 550                 555


<210>    7
<211>    1677
<212>    DNA
<213>    PHA synthase from Pseudomonas aeruginosa PAO1 KCTC 1637 (phaC1Pae)

<400>    7
atgagtcaga agaacaataa cgagcttccc aagcaagccg cggaaaacac gctgaacctg        60

aatccggtga tcggcatccg gggcaaggac ctgctcacct ccgcgcgcat ggtcctgctc       120

caggcggtgc gccagccgct gcacagcgcc aggcacgtgg cgcatttcag cctggagctg       180

aagaacgtcc tgctcggcca gtcggagcta cgcccaggcg atgacgaccg acgcttttcc       240

gatccggcct ggagccagaa tccactgtac aagcgctaca tgcagaccta cctggcctgg       300

cgcaaggagc tgcacagctg gatcagccac agcgacctgt cgccgcagga catcagtcgt       360

ggccagttcg tcatcaacct gctgaccgag gcgatgtcgc cgaccaacag cctgagcaac       420

ccggcggcgg tcaagcgctt cttcgagacc ggcggcaaga gcctgctgga cggcctcggc       480

cacctggcca aggacctggt gaacaacggc gggatgccga gccaggtgga catggacgcc       540

ttcgaggtgg gcaagaacct ggccaccacc gagggcgccg tggtgttccg caacgacgtg       600

ctggaactga tccagtaccg gccgatcacc gagtcggtgc acgaacgccc gctgctggtg       660

gtgccgccgc agatcaacaa gttctacgtc ttcgacctgt cgccggacaa gagcctggcg       720

cgcttctgcc tgcgcaacgg cgtgcagacc ttcatcgtca gttggcgcaa cccgaccaag       780

tcgcagcgcg aatggggcct gaccacctat atcgaggcgc tcaaggaggc catcgaggta       840

gtcctgtcga tcaccggcag caaggacctc aacctcctcg gcgcctgctc cggcgggatc       900

accaccgcga ccctggtcgg ccactacgtg gccagcggcg agaagaaggt caacgccttc       960

acccaactgg tcagcgtgct cgacttcgaa ctgaataccc aggtcgcgct gttcgccgac      1020

gagaagactc tggaggccgc caagcgtcgt tcctaccagt ccggcgtgct ggagggcaag      1080

gacatggcca aggtgttcgc ctggatgcgc cccaacgacc tgatctggaa ctactgggtc      1140

aacaactacc tgctcggcaa ccagccgccg gcgttcgaca tcctctactg gaacaacgac      1200

accacgcgcc tgcccgccgc gctgcacggc gagttcgtcg aactgttcaa gagcaacccg 1260

ctgaaccgcc ccggcgccct ggaggtctcc ggcacgccca tcgacctgaa gcaggtgact 1320

tgcgacttct actgtgtcgc cggtctgaac gaccacatca ccccctggga gtcgtgctac 1380

aagtcggcca ggctgctggg tggcaagtgc gagttcatcc tctccaacag cggtcacatc 1440

cagagcatcc tcaacccacc gggcaacccc aaggcacgct tcatgaccaa tccggaactg 1500

cccgccgagc ccaaggcctg gctggaacag gccggcaagc acgccgactc gtggtggttg 1560

cactggcagc aatggctggc cgaacgctcc ggcaagaccc gcaaggcgcc cgccagcctg 1620

ggcaacaaga cctatccggc cggcgaagcc gcgcccggaa cctacgtgca tgaacga 1677

<210> 8
<211> 559
<212> PRT
<213> PHA synthase from Pseudomonas aeruginosa PAO1 KCTC 1637 (PhaC1Pae)

<400> 8
Met Ser Gln Lys Asn Asn Asn Glu Leu Pro Lys Gln Ala Ala Glu Asn
1               5                   10                  15

Thr Leu Asn Leu Asn Pro Val Ile Gly Ile Arg Gly Lys Asp Leu Leu
            20                  25                  30

Thr Ser Ala Arg Met Val Leu Leu Gln Ala Val Arg Gln Pro Leu His
        35                  40                  45

Ser Ala Arg His Val Ala His Phe Ser Leu Glu Leu Lys Asn Val Leu
    50                  55                  60

Leu Gly Gln Ser Glu Leu Arg Pro Gly Asp Asp Asp Arg Arg Phe Ser
65                  70                  75                  80

Asp Pro Ala Trp Ser Gln Asn Pro Leu Tyr Lys Arg Tyr Met Gln Thr
                85                  90                  95

Tyr Leu Ala Trp Arg Lys Glu Leu His Ser Trp Ile Ser His Ser Asp
            100                 105                 110

Leu Ser Pro Gln Asp Ile Ser Arg Gly Gln Phe Val Ile Asn Leu Leu
        115                 120                 125

Thr Glu Ala Met Ser Pro Thr Asn Ser Leu Ser Asn Pro Ala Ala Val
    130                 135                 140

Lys Arg Phe Phe Glu Thr Gly Gly Lys Ser Leu Leu Asp Gly Leu Gly
145                 150                 155                 160

His Leu Ala Lys Asp Leu Val Asn Asn Gly Gly Met Pro Ser Gln Val
                165                 170                 175

Asp Met Asp Ala Phe Glu Val Gly Lys Asn Leu Ala Thr Thr Glu Gly
            180                 185                 190

Ala Val Val Phe Arg Asn Asp Val Leu Glu Leu Ile Gln Tyr Arg Pro
        195                 200                 205

Ile Thr Glu Ser Val His Glu Arg Pro Leu Leu Val Val Pro Pro Gln
    210                 215                 220

Ile Asn Lys Phe Tyr Val Phe Asp Leu Ser Pro Asp Lys Ser Leu Ala
225                 230                 235                 240

Arg Phe Cys Leu Arg Asn Gly Val Gln Thr Phe Ile Val Ser Trp Arg
                245                 250                 255

Asn Pro Thr Lys Ser Gln Arg Glu Trp Gly Leu Thr Thr Tyr Ile Glu
            260                 265                 270

Ala Leu Lys Glu Ala Ile Glu Val Val Leu Ser Ile Thr Gly Ser Lys
        275                 280                 285

Asp Leu Asn Leu Leu Gly Ala Cys Ser Gly Gly Ile Thr Thr Ala Thr
    290                 295                 300

Leu Val Gly His Tyr Val Ala Ser Gly Glu Lys Lys Val Asn Ala Phe
305                 310                 315                 320

Thr Gln Leu Val Ser Val Leu Asp Phe Glu Leu Asn Thr Gln Val Ala
                325                 330                 335

Leu Phe Ala Asp Glu Lys Thr Leu Glu Ala Ala Lys Arg Arg Ser Tyr
            340                 345                 350

Gln Ser Gly Val Leu Glu Gly Lys Asp Met Ala Lys Val Phe Ala Trp
        355                 360                 365

Met Arg Pro Asn Asp Leu Ile Trp Asn Tyr Trp Val Asn Asn Tyr Leu
    370                 375                 380

Leu Gly Asn Gln Pro Pro Ala Phe Asp Ile Leu Tyr Trp Asn Asn Asp
385                 390                 395                 400

Thr Thr Arg Leu Pro Ala Ala Leu His Gly Glu Phe Val Glu Leu Phe
                405                 410                 415

Lys Ser Asn Pro Leu Asn Arg Pro Gly Ala Leu Glu Val Ser Gly Thr
            420                 425                 430

Pro Ile Asp Leu Lys Gln Val Thr Cys Asp Phe Tyr Cys Val Ala Gly
        435                 440                 445

Leu Asn Asp His Ile Thr Pro Trp Glu Ser Cys Tyr Lys Ser Ala Arg
    450                 455                 460

Leu Leu Gly Gly Lys Cys Glu Phe Ile Leu Ser Asn Ser Gly His Ile
465                 470                 475                 480

Gln Ser Ile Leu Asn Pro Pro Gly Asn Pro Lys Ala Arg Phe Met Thr
                485                 490                 495

Asn Pro Glu Leu Pro Ala Glu Pro Lys Ala Trp Leu Glu Gln Ala Gly
            500                 505                 510

Lys His Ala Asp Ser Trp Trp Leu His Trp Gln Gln Trp Leu Ala Glu
        515                 520                 525

Arg Ser Gly Lys Thr Arg Lys Ala Pro Ala Ser Leu Gly Asn Lys Thr
    530                 535                 540

Tyr Pro Ala Gly Glu Ala Ala Pro Gly Thr Tyr Val His Glu Arg
545                 550                 555

<210>   9

<211>    26
<212>    DNA
<213>    Artificial Sequence

<220>
<223>    primer for recognition site insertion


<400>    9
gcaatgcccg gagccgggct agctag                                            26


<210>    10
<211>    42
<212>    DNA
<213>    Artificial Sequence

<220>
<223>    primer for recognition site insertion


<400>    10
gtcatcgtta ttcttgttac tcatgatttg attgtctctc tg                          42


<210>    11
<211>    42
<212>    DNA
<213>    Artificial Sequence

<220>
<223>    primer for PHA synthase cloning


<400>    11
cagagagaca atcaaatcat gagtaacaag aataacgatg ac                          42


<210>    12
<211>    39
<212>    DNA
<213>    Artificial Sequence

<220>
<223>    primer for PHA synthase cloning


<400>    12
gcactcatgc aagcgttaac gttcatggac ataagtacc                              39


<210>    13
<211>    39
<212>    DNA
<213>    Artificial Sequence

<220>
<223>    primer for recognition site insertion


<400>    13
ggtacttatg tccatgaacg ttaacgcttg catgagtgc                              39


<210>    14

<211>    42
<212>    DNA
<213>    Artificial Sequence

<220>
<223>    primer for recognition site insertion


<400>    14
ctcatcgttg ttcttgttac tcatgatttg attgtctctc tg                          42


<210>    15
<211>    42
<212>    DNA
<213>    Artificial Sequence

<220>
<223>    primer for PHA synthase cloning


<400>    15
cagagagaca atcaaatcat gagtaacaag aacaacgatg ag                          42


<210>    16
<211>    37
<212>    DNA
<213>    Artificial Sequence

<220>
<223>    primer for PHA synthase cloning


<400>    16
gcactcatgc aagcgtcaac gctcgtgaac gtaggtg                               37


<210>    17
<211>    37
<212>    DNA
<213>    Artificial Sequence

<220>
<223>    primer for recognition site insertion


<400>    17
cacctacgtt cacgagcgtt gacgcttgca tgagtgc                               37


<210>    18
<211>    42
<212>    DNA
<213>    Artificial Sequence

<220>
<223>    primer for recognition site insertion


<400>    18
gtcttcattg ttcttgttgc tcatgatttg attgtctctc tg                          42


<210>    19

<211>    42
<212>    DNA
<213>    Artificial Sequence

<220>
<223>    primer for PHA synthase cloning


<400>    19
cagagagaca atcaaatcat gagcaacaag aacaatgaag ac                          42


<210>    20
<211>    37
<212>    DNA
<213>    Artificial Sequence

<220>
<223>    primer for PHA synthase cloning


<400>    20
gcactcatgc aagcgtcatc gctcgtgcac ataggtg                                37


<210>    21
<211>    37
<212>    DNA
<213>    Artificial Sequence

<220>
<223>    primer for recognition site insertion


<400>    21
cacctatgtg cacgagcgat gacgcttgca tgagtgc                                37


<210>    22
<211>    42
<212>    DNA
<213>    Artificial Sequence

<220>
<223>    primer for recognition site insertion


<400>    22
ctcgttattg ttcttctgac tcatgatttg attgtctctc tg                          42


<210>    23
<211>    42
<212>    DNA
<213>    Artificial Sequence

<220>
<223>    primer for PHA synthase cloning


<400>    23
cagagagaca atcaaatcat gagtcagaag aacaataacg ag                          42


<210>    24

<211>    38
<212>    DNA
<213>    Artificial Sequence

<220>
<223>    primer for PHA synthase cloning


<400>    24
gcactcatgc aagcgtcatc gttcatgcac gtaggttc                              38


<210>    25
<211>    38
<212>    DNA
<213>    Artificial Sequence

<220>
<223>    primer for recognition site insertion


<400>    25
gaacctacgt gcatgaacga tgacgcttgc atgagtgc                              38


<210>    26
<211>    22
<212>    DNA
<213>    Artificial Sequence

<220>
<223>    primer for recognition site insertion


<400>    26
gaaattgtta tccgcctgca gg                                               22


<210>    27
<211>    33
<212>    DNA
<213>    Artificial Sequence

<220>
<223>    primer for E130D mutation in PhaC1Ps6-19


<400>    27
atcaacctca tgaccgatgc gatggcgccg acc                                   33


<210>    28
<211>    33
<212>    DNA
<213>    Artificial Sequence

<220>
<223>    primer for E130D mutation in PhaC1Ps6-19


<400>    28
ggtcggcgcc atcgcatcgg tcatgaggtt gat                                   33


<210>    29

<211>    33
<212>    DNA
<213>    Artificial Sequence

<220>
<223>    primer for S325T mutation in PhaC1Ps6-19


<400>    29
ctgaccttgc tggtgaccgt gcttgatacc acc                                    33


<210>    30
<211>    33
<212>    DNA
<213>    Artificial Sequence

<220>
<223>    primer for S325T mutation in PhaC1Ps6-19


<400>    30
ggtggtatca agcacggtca ccagcaaggt cag                                    33


<210>    31
<211>    30
<212>    DNA
<213>    Artificial Sequence

<220>
<223>    primer for S477G mutation in PhaC1Ps6-19


<400>    31
gaattcgtgc tgtcgagcgg cgggcatatc                                        30


<210>    32
<211>    30
<212>    DNA
<213>    Artificial Sequence

<220>
<223>    primer for S477G mutation in PhaC1Ps6-19


<400>    32
gatatgcccg ccgctcgaca gcacgaattc                                        30


<210>    33
<211>    28
<212>    DNA
<213>    Artificial Sequence

<220>
<223>    primer for Q481K mutation in PhaC1Ps6-19


<400>    33
gggcatatca aaagcatcct gaacccgc                                          28


<210>    34

<211>    28
<212>    DNA
<213>    Artificial Sequence

<220>
<223>    primer for Q481K mutation in PhaC1Ps6-19


<400>    34
gcgggttcag gatgcttttg atatgccc                                          28


<210>    35
<211>    33
<212>    DNA
<213>    Artificial Sequence

<220>
<223>    primer for E130D mutation in PhaC1Pch


<400>    35
atcaacctga tgaccgatgc catggcgccg acc                                    33


<210>    36
<211>    33
<212>    DNA
<213>    Artificial Sequence

<220>
<223>    primer for E130D mutation in PhaC1Pch


<400>    36
ggtcggcgcc atggcatcgg tcatcaggtt gat                                    33


<210>    37
<211>    28
<212>    DNA
<213>    Artificial Sequence

<220>
<223>    primer for Q481K mutation in PhaC1Pch


<400>    37
gggcatatca aaagcatcct caacccgc                                          28


<210>    38
<211>    28
<212>    DNA
<213>    Artificial Sequence

<220>
<223>    primer for Q481K mutation in PhaC1Pch


<400>    38
gcgggttgag gatgcttttg atatgccc                                          28


<210>    39

<211>    30
<212>    DNA
<213>    Artificial Sequence

<220>
<223>    primer for S477F mutation in PhaC1Pch


<400>    39
gaattcgtcc tctccagctt tgggcatatc                                        30


<210>    40
<211>    30
<212>    DNA
<213>    Artificial Sequence

<220>
<223>    primer for S477F mutation in PhaC1Pch


<400>    40
gatatgccca aagctggaga ggacgaattc                                        30


<210>    41
<211>    33
<212>    DNA
<213>    Artificial Sequence

<220>
<223>    primer for S325T mutation in PhaC1Pch


<400>    41
ctgaccctgc tggtcaccgt gctcgatacc acc                                    33


<210>    42
<211>    33
<212>    DNA
<213>    Artificial Sequence

<220>
<223>    primer for S325T mutation in PhaC1Pch


<400>    42
ggtggtatcg agcacggtga ccagcagggt cag                                    33


<210>    43
<211>    30
<212>    DNA
<213>    Artificial Sequence

<220>
<223>    primer for S477G mutation in PhaC1Pch


<400>    43
gaattcgtcc tctccagcgg cgggcatatc                                        30


<210>    44

<211>    30
<212>    DNA
<213>    Artificial Sequence

<220>
<223>    primer for S477G mutation in PhaC1Pch


<400>    44
gatatgcccg ccgctggaga ggacgaattc                                        30


<210>    45
<211>    33
<212>    DNA
<213>    Artificial Sequence

<220>
<223>    primer for E130D mutation in PhaC1Ppu


<400>    45
atcaacctga tgaccgatgc catggcgccg acc                                    33


<210>    46
<211>    33
<212>    DNA
<213>    Artificial Sequence

<220>
<223>    primer for E130D mutation in PhaC1Ppu


<400>    46
ggtcggcgcc atggcatcgg tcatcaggtt gat                                    33


<210>    47
<211>    28
<212>    DNA
<213>    Artificial Sequence

<220>
<223>    primer for Q481K mutation in PhaC1Ppu


<400>    47
gggcatatca aaagcatcct caacccgc                                          28


<210>    48
<211>    28
<212>    DNA
<213>    Artificial Sequence

<220>
<223>    primer for Q481K mutation in PhaC1Ppu


<400>    48
gcgggttgag gatgcttttg atatgccc                                          28


<210>    49

<211>    30
<212>    DNA
<213>    Artificial Sequence

<220>
<223>    primer for S477F mutation in PhaC1Ppu


<400>    49
gaattcgtac tgtccaactt tgggcatatc                                        30


<210>    50
<211>    30
<212>    DNA
<213>    Artificial Sequence

<220>
<223>    primer for S477F mutation in PhaC1Ppu


<400>    50
gatatgccca aagttggaca gtacgaattc                                        30


<210>    51
<211>    33
<212>    DNA
<213>    Artificial Sequence

<220>
<223>    primer for S325T mutation in PhaC1Ppu


<400>    51
ctgaccctgc tggtcaccgt gctggacacc acc                                    33


<210>    52
<211>    33
<212>    DNA
<213>    Artificial Sequence

<220>
<223>    primer for S325T mutation in PhaC1Ppu


<400>    52
ggtggtgtcc agcacggtga ccagcagggt cag                                    33


<210>    53
<211>    30
<212>    DNA
<213>    Artificial Sequence

<220>
<223>    primer for S477G mutation in PhaC1Ppu


<400>    53
gaattcgtac tgtccaacgg cgggcatatc                                        30


<210>    54

<211>    30
<212>    DNA
<213>    Artificial Sequence

<220>
<223>    primer for S477G mutation in PhaC1Ppu


<400>    54
gatatgcccg ccgttggaca gtacgaattc                                        30


<210>    55
<211>    33
<212>    DNA
<213>    Artificial Sequence

<220>
<223>    primer for E130D mutation in PhaC1Pre


<400>    55
atcaacctga tgaccgatgc gatggcgccc acc                                    33


<210>    56
<211>    33
<212>    DNA
<213>    Artificial Sequence

<220>
<223>    primer for E130D mutation in PhaC1Pre


<400>    56
ggtgggcgcc atcgcatcgg tcatcaggtt gat                                    33


<210>    57
<211>    28
<212>    DNA
<213>    Artificial Sequence

<220>
<223>    primer for Q481K mutation in PhaC1Pre


<400>    57
ggccacatca aaagcattct caacccac                                          28


<210>    58
<211>    28
<212>    DNA
<213>    Artificial Sequence

<220>
<223>    primer for Q481K mutation in PhaC1Pre


<400>    58
gtgggttgag aatgcttttg atgtggcc                                          28


<210>    59

<211>    30
<212>    DNA
<213>    Artificial Sequence

<220>
<223>    primer for S477F mutation in PhaC1Pre


<400>    59
gagttcgtgc tgtccaactt tggccacatc                                        30


<210>    60
<211>    30
<212>    DNA
<213>    Artificial Sequence

<220>
<223>    primer for S477F mutation in PhaC1Pre


<400>    60
gatgtggcca aagttggaca gcacgaactc                                        30


<210>    61
<211>    33
<212>    DNA
<213>    Artificial Sequence

<220>
<223>    primer for S325T mutation in PhaC1Pre


<400>    61
ttcacccaga tggtcaccgt gctcgacttc aac                                    33


<210>    62
<211>    33
<212>    DNA
<213>    Artificial Sequence

<220>
<223>    primer for S325T mutation in PhaC1Pre


<400>    62
gttgaagtcg agcacggtga ccatctgggt gaa                                    33


<210>    63
<211>    30
<212>    DNA
<213>    Artificial Sequence

<220>
<223>    primer for S477G mutation in PhaC1Pre


<400>    63
gagttcgtgc tgtccaacgg cggccacatc                                        30


<210>    64

<211>    30
<212>    DNA
<213>    Artificial Sequence

<220>
<223>    primer for S477G mutation in PhaC1Pre


<400>    64
gatgtggccg ccgttggaca gcacgaactc                                        30


<210>    65
<211>    33
<212>    DNA
<213>    Artificial Sequence

<220>
<223>    primer for E130D mutation in PhaC1Pae


<400>    65
atcaacctgc tgaccgatgc gatgtcgccg acc                                    33


<210>    66
<211>    33
<212>    DNA
<213>    Artificial Sequence

<220>
<223>    primer for E130D mutation in PhaC1Pae


<400>    66
ggtcggcgac atcgcatcgg tcagcaggtt gat                                    33


<210>    67
<211>    28
<212>    DNA
<213>    Artificial Sequence

<220>
<223>    primer for Q481K mutation in PhaC1Pae


<400>    67
ggtcacatca aaagcatcct caacccac                                          28


<210>    68
<211>    28
<212>    DNA
<213>    Artificial Sequence

<220>
<223>    primer for Q481K mutation in PhaC1Pae


<400>    68
gtgggttgag gatgcttttg atgtgacc                                          28


<210>    69

<211>    30
<212>    DNA
<213>    Artificial Sequence

<220>
<223>    primer for S477F mutation in PhaC1Pae


<400>    69
gagttcatcc tctccaactt tggtcacatc                                        30


<210>    70
<211>    30
<212>    DNA
<213>    Artificial Sequence

<220>
<223>    primer for S477F mutation in PhaC1Pae


<400>    70
gatgtgacca aagttggaga ggatgaactc                                        30


<210>    71
<211>    33
<212>    DNA
<213>    Artificial Sequence

<220>
<223>    primer for S325T mutation in PhaC1Pae


<400>    71
ttcacccaac tggtcaccgt gctcgacttc gaa                                    33


<210>    72
<211>    33
<212>    DNA
<213>    Artificial Sequence

<220>
<223>    primer for S325T mutation in PhaC1Pae


<400>    72
ttcgaagtcg agcacggtga ccagttgggt gaa                                    33


<210>    73
<211>    30
<212>    DNA
<213>    Artificial Sequence

<220>
<223>    primer for S477G mutation in PhaC1Pae


<400>    73
gagttcatcc tctccaacgg cggtcacatc                                        30


<210>    74

<211>    30
<212>    DNA
<213>    Artificial Sequence

<220>
<223>    primer for S477G mutation in PhaC1Pae


<400>    74
gatgtgaccg ccgttggaga ggatgaactc                                        30


<210>    75
<211>    1677
<212>    DNA
<213>    PHA synthase from Pseudomonas sp. MBEL 6-19 KCTC 11027BP
(phaC1Ps6-19)

<400>    75
atgagtaaca agagtaacga tgagttgaag tatcaagcct ctgaaaacac cttggggctt      60

aatcctgtcg ttgggctgcg tggaaaggat ctactggctt ctgctcgaat ggtgcttagg     120

caggccatca agcaaccggt gcacagcgtc aaacatgtcg cgcactttgg tcttgaactc     180

aagaacgtac tgctgggtaa atccgggctg caaccgacca gcgatgaccg tcgcttcgcc     240

gatccggcct ggagccagaa cccgctctat aaacgttatt tgcaaaccta cctggcgtgg     300

cgcaaggaac tccacgactg gatcgatgaa agtaacctcg cccccaagga tgtggcgcgt     360

gggcacttcg tgatcaacct catgaccgaa gcgatggcgc cgaccaacac cgcggccaac     420

ccggcggcag tcaaacgctt ttttgaaacc ggtggcaaaa gcctgctcga cggcctctcg     480

cacctggcca aggatctggt acacaacggc ggcatgccga gccaggtcaa catgggtgca     540

ttcgaggtcg gcaagagcct gggcgtgacc gaaggcgcgg tggtgtttcg caacgatgtg     600

ctggaactga tccagtacaa gccgaccacc gagcaggtat acgaacgccc gctgctggtg     660

gtgccgccgc agatcaacaa gttctacgtt ttcgacctga gcccggacaa gagcctggcg     720

cggttctgcc tgcgcaacaa cgtgcaaacg ttcatcgtca gctggcgaaa tcccaccaag     780

gaacagcgag agtggggcct gtcgacctac atcgaagccc tcaaggaagc ggttgacgtc     840

gttaccgcga tcaccggcag caaagacgtg aacatgctcg gggcctgctc cggcggcatc     900

acttgcactg cgctgctggg ccattacgcg gcgattggcg aaaacaaggt caacgccctg     960

accttgctgg tgagcgtgct tgataccacc ctcgacagcg acgtcgccct gttcgtcaat    1020

gaacagaccc ttgaagccgc caagcgccac tcgtaccagg ccggcgtact ggaaggccgc    1080

gacatggcga aggtcttcgc ctggatgcgc cccaacgatc tgatctggaa ctactgggtc    1140

aacaattacc tgctaggcaa cgaaccgccg gtgttcgaca tcctgttctg gaacaacgac    1200

accacacggt tgcccgcggc gttccacggc gacctgatcg aactgttcaa aaataaccca    1260

ctgattcgcc cgaatgcact ggaagtgtgc ggcaccccca tcgacctcaa gcaggtgacg    1320

gccgacatct tttccctggc cggcaccaac gaccacatca ccccgtggaa gtcctgctac    1380

aagtcggcgc aactgtttgg cggcaacgtt gaattcgtgc tgtcgagcag cgggcatatc 1440

cagagcatcc tgaacccgcc gggcaatccg aaatcgcgct acatgaccag caccgaagtg 1500

gcggaaaatg ccgatgaatg gcaagcgaat gccaccaagc atacagattc ctggtggctg 1560

cactggcagg cctggcaggc ccaacgctcg ggcgagctga aaaagtcccc gacaaaactg 1620

ggcagcaagg cgtatccggc aggtgaagcg gcgccaggca cgtacgtgca cgaacgg 1677

<210> 76
<211> 559
<212> PRT
<213> PHA synthase from Pseudomonas sp. MBEL 6-19 KCTC 11027BP
(PhaC1Ps6-19)

<400> 76
Met Ser Asn Lys Ser Asn Asp Glu Leu Lys Tyr Gln Ala Ser Glu Asn
1               5                   10                  15

Thr Leu Gly Leu Asn Pro Val Val Gly Leu Arg Gly Lys Asp Leu Leu
            20                  25                  30

Ala Ser Ala Arg Met Val Leu Arg Gln Ala Ile Lys Gln Pro Val His
        35                  40                  45

Ser Val Lys His Val Ala His Phe Gly Leu Glu Leu Lys Asn Val Leu
    50                  55                  60

Leu Gly Lys Ser Gly Leu Gln Pro Thr Ser Asp Asp Arg Arg Phe Ala
65                  70                  75                  80

Asp Pro Ala Trp Ser Gln Asn Pro Leu Tyr Lys Arg Tyr Leu Gln Thr
                85                  90                  95

Tyr Leu Ala Trp Arg Lys Glu Leu His Asp Trp Ile Asp Glu Ser Asn
            100                 105                 110

Leu Ala Pro Lys Asp Val Ala Arg Gly His Phe Val Ile Asn Leu Met
        115                 120                 125

Thr Glu Ala Met Ala Pro Thr Asn Thr Ala Ala Asn Pro Ala Ala Val
    130                 135                 140

Lys Arg Phe Phe Glu Thr Gly Gly Lys Ser Leu Leu Asp Gly Leu Ser
145                 150                 155                 160

His Leu Ala Lys Asp Leu Val His Asn Gly Gly Met Pro Ser Gln Val
                165                 170                 175

Asn Met Gly Ala Phe Glu Val Gly Lys Ser Leu Gly Val Thr Glu Gly
            180                 185                 190

Ala Val Val Phe Arg Asn Asp Val Leu Glu Leu Ile Gln Tyr Lys Pro
        195                 200                 205

Thr Thr Glu Gln Val Tyr Glu Arg Pro Leu Leu Val Val Pro Pro Gln
    210                 215                 220

Ile Asn Lys Phe Tyr Val Phe Asp Leu Ser Pro Asp Lys Ser Leu Ala
225                 230                 235                 240

Arg Phe Cys Leu Arg Asn Asn Val Gln Thr Phe Ile Val Ser Trp Arg
                245                 250                 255

Asn Pro Thr Lys Glu Gln Arg Glu Trp Gly Leu Ser Thr Tyr Ile Glu
            260                 265                 270

Ala Leu Lys Glu Ala Val Asp Val Val Thr Ala Ile Thr Gly Ser Lys
        275                 280                 285

Asp Val Asn Met Leu Gly Ala Cys Ser Gly Gly Ile Thr Cys Thr Ala
    290                 295                 300

Leu Leu Gly His Tyr Ala Ala Ile Gly Glu Asn Lys Val Asn Ala Leu
305                 310                 315                 320

Thr Leu Leu Val Ser Val Leu Asp Thr Thr Leu Asp Ser Asp Val Ala
                325                 330                 335

Leu Phe Val Asn Glu Gln Thr Leu Glu Ala Ala Lys Arg His Ser Tyr
            340                 345                 350

Gln Ala Gly Val Leu Glu Gly Arg Asp Met Ala Lys Val Phe Ala Trp
        355                 360                 365

Met Arg Pro Asn Asp Leu Ile Trp Asn Tyr Trp Val Asn Asn Tyr Leu
    370                 375                 380

Leu Gly Asn Glu Pro Pro Val Phe Asp Ile Leu Phe Trp Asn Asn Asp
385                 390                 395                 400

Thr Thr Arg Leu Pro Ala Ala Phe His Gly Asp Leu Ile Glu Leu Phe
                405                 410                 415

Lys Asn Asn Pro Leu Ile Arg Pro Asn Ala Leu Glu Val Cys Gly Thr
            420                 425                 430

Pro Ile Asp Leu Lys Gln Val Thr Ala Asp Ile Phe Ser Leu Ala Gly
        435                 440                 445

Thr Asn Asp His Ile Thr Pro Trp Lys Ser Cys Tyr Lys Ser Ala Gln
    450                 455                 460

Leu Phe Gly Gly Asn Val Glu Phe Val Leu Ser Ser Ser Gly His Ile
465                 470                 475                 480

Gln Ser Ile Leu Asn Pro Pro Gly Asn Pro Lys Ser Arg Tyr Met Thr
                485                 490                 495

Ser Thr Glu Val Ala Glu Asn Ala Asp Glu Trp Gln Ala Asn Ala Thr
            500                 505                 510

Lys His Thr Asp Ser Trp Trp Leu His Trp Gln Ala Trp Gln Ala Gln
        515                 520                 525

Arg Ser Gly Glu Leu Lys Lys Ser Pro Thr Lys Leu Gly Ser Lys Ala
    530                 535                 540

Tyr Pro Ala Gly Glu Ala Ala Pro Gly Thr Tyr Val His Glu Arg
545                 550                 555


<210>    77
<211>    1572
<212>    DNA
<213>    Propionyl-CoA transferase from Clostridium propionicum (pctCp)

<400>    77

atgagaaagg ttcccattat taccgcagat gaggctgcaa agcttattaa agacggtgat 60

acagttacaa caagtggttt cgttggaaat gcaatccctg aggctcttga tagagctgta 120

gaaaaaagat tcttagaaac aggcgaaccc aaaaacatta cctatgttta ttgtggttct 180

caaggtaaca gagacggaag aggtgctgag cactttgctc atgaaggcct tttaaaacgt 240

tacatcgctg gtcactgggc tacagttcct gctttgggta aaatggctat ggaaaataaa 300

atggaagcat ataatgtatc tcagggtgca ttgtgtcatt tgttccgtga tatagcttct 360

cataagccag gcgtatttac aaaggtaggt atcggtactt tcattgaccc cagaaatggc 420

ggcggtaaag taaatgatat taccaaagaa gatattgttg aattggtaga gattaagggt 480

caggaatatt tattctaccc tgcttttcct attcatgtag ctcttattcg tggtacttac 540

gctgatgaaa gcggaaatat cacatttgag aaagaagttg ctcctctgga aggaacttca 600

gtatgccagg ctgttaaaaa cagtggcggt atcgttgtag ttcaggttga aagagtagta 660

aaagctggta ctcttgaccc tcgtcatgta aaagttccag gaatttatgt tgactatgtt 720

gttgttgctg acccagaaga tcatcagcaa tctttagatt gtgaatatga tcctgcatta 780

tcaggcgagc atagaagacc tgaagttgtt ggagaaccac ttcctttgag tgcaaagaaa 840

gttattggtc gtcgtggtgc cattgaatta gaaaaagatg ttgctgtaaa tttaggtgtt 900

ggtgcgcctg aatatgtagc aagtgttgct gatgaagaag gtatcgttga ttttatgact 960

ttaactgctg aaagtggtgc tattggtggt gttcctgctg gtggcgttcg ctttggtgct 1020

tcttataatg cggatgcatt gatcgatcaa ggttatcaat tcgattacta tgatggcggc 1080

ggcttagacc tttgctattt aggcttagct gaatgcgatg aaaaaggcaa tatcaacgtt 1140

tcaagatttg gccctcgtat cgctggttgt ggtggtttca tcaacattac acagaataca 1200

cctaaggtat tcttctgtgg tactttcaca gcaggtggct taaaggttaa aattgaagat 1260

ggcaaggtta ttattgttca agaaggcaag cagaaaaaat tcttgaaagc tgttgagcag 1320

attacattca atggtgacgt tgcacttgct aataagcaac aagtaactta tattacagaa 1380

agatgcgtat tccttttgaa ggaagatggt ttgcacttat ctgaaattgc acctggtatt 1440

gatttgcaga cacagattct tgacgttatg gattttgcac ctattattga cagagatgca 1500

aacggccaaa tcaaattgat ggacgctgct ttgtttgcag aaggcttaat gggtctgaag 1560

gaaatgaagt cc 1572

<210> 78
<211> 524
<212> PRT
<213> Propionyl-CoA transferase from Clostridium propionicum (PctCp)

<400> 78
Met Arg Lys Val Pro Ile Ile Thr Ala Asp Glu Ala Ala Lys Leu Ile
1               5                   10                  15

Lys Asp Gly Asp Thr Val Thr Thr Ser Gly Phe Val Gly Asn Ala Ile

            20                  25                  30

Pro Glu Ala Leu Asp Arg Ala Val Glu Lys Arg Phe Leu Glu Thr Gly
        35                  40                  45

Glu Pro Lys Asn Ile Thr Tyr Val Tyr Cys Gly Ser Gln Gly Asn Arg
    50                  55                  60

Asp Gly Arg Gly Ala Glu His Phe Ala His Glu Gly Leu Leu Lys Arg
65                  70                  75                  80

Tyr Ile Ala Gly His Trp Ala Thr Val Pro Ala Leu Gly Lys Met Ala
                85                  90                  95

Met Glu Asn Lys Met Glu Ala Tyr Asn Val Ser Gln Gly Ala Leu Cys
            100                 105                 110

His Leu Phe Arg Asp Ile Ala Ser His Lys Pro Gly Val Phe Thr Lys
        115                 120                 125

Val Gly Ile Gly Thr Phe Ile Asp Pro Arg Asn Gly Gly Gly Lys Val
    130                 135                 140

Asn Asp Ile Thr Lys Glu Asp Ile Val Glu Leu Val Glu Ile Lys Gly
145                 150                 155                 160

Gln Glu Tyr Leu Phe Tyr Pro Ala Phe Pro Ile His Val Ala Leu Ile
                165                 170                 175

Arg Gly Thr Tyr Ala Asp Glu Ser Gly Asn Ile Thr Phe Glu Lys Glu
            180                 185                 190

Val Ala Pro Leu Glu Gly Thr Ser Val Cys Gln Ala Val Lys Asn Ser
        195                 200                 205

Gly Gly Ile Val Val Val Gln Val Glu Arg Val Val Lys Ala Gly Thr
    210                 215                 220

Leu Asp Pro Arg His Val Lys Val Pro Gly Ile Tyr Val Asp Tyr Val
225                 230                 235                 240

Val Val Ala Asp Pro Glu Asp His Gln Gln Ser Leu Asp Cys Glu Tyr
                245                 250                 255

Asp Pro Ala Leu Ser Gly Glu His Arg Arg Pro Glu Val Val Gly Glu
            260                 265                 270

Pro Leu Pro Leu Ser Ala Lys Lys Val Ile Gly Arg Arg Gly Ala Ile
        275                 280                 285

Glu Leu Glu Lys Asp Val Ala Val Asn Leu Gly Val Gly Ala Pro Glu
    290                 295                 300

Tyr Val Ala Ser Val Ala Asp Glu Glu Gly Ile Val Asp Phe Met Thr
305                 310                 315                 320

Leu Thr Ala Glu Ser Gly Ala Ile Gly Gly Val Pro Ala Gly Gly Val
                325                 330                 335

Arg Phe Gly Ala Ser Tyr Asn Ala Asp Ala Leu Ile Asp Gln Gly Tyr
            340                 345                 350

Gln Phe Asp Tyr Tyr Asp Gly Gly Gly Leu Asp Leu Cys Tyr Leu Gly
        355                 360                 365

Leu Ala Glu Cys Asp Glu Lys Gly Asn Ile Asn Val Ser Arg Phe Gly
    370                 375                 380

Pro Arg Ile Ala Gly Cys Gly Gly Phe Ile Asn Ile Thr Gln Asn Thr
385                 390                 395                 400

Pro Lys Val Phe Phe Cys Gly Thr Phe Thr Ala Gly Gly Leu Lys Val
                405                 410                 415

Lys Ile Glu Asp Gly Lys Val Ile Ile Val Gln Glu Gly Lys Gln Lys
            420                 425                 430

Lys Phe Leu Lys Ala Val Glu Gln Ile Thr Phe Asn Gly Asp Val Ala
        435                 440                 445

Leu Ala Asn Lys Gln Gln Val Thr Tyr Ile Thr Glu Arg Cys Val Phe
    450                 455                 460

Leu Leu Lys Glu Asp Gly Leu His Leu Ser Glu Ile Ala Pro Gly Ile
465                 470                 475                 480

Asp Leu Gln Thr Gln Ile Leu Asp Val Met Asp Phe Ala Pro Ile Ile
                485                 490                 495

Asp Arg Asp Ala Asn Gly Gln Ile Lys Leu Met Asp Ala Ala Leu Phe
            500                 505                 510

Ala Glu Gly Leu Met Gly Leu Lys Glu Met Lys Ser
        515                 520